# EUROPEAN PATENT APPLICATION

(11) **EP 4 364 736 A1**
(43) Date of publication of application: **08.05.2024**
(21) Application number: 22845341.1
(22) Date of filing: 20.07.2022
(51) Int. Cl.: A61K 31/4375, A61P 27/10

(54) **METHOD FOR TREATING MYOPIA WITH VINPOCETINE**

(30) Priority: 20.07.2021 CN 202110819425
(71) Applicant: The Eye Hospital of Wenzhou Medical University, Wenzhou, Zhejiang 325000 (CN)
(72) Inventor: ZHOU, Xiangtian, Wenzhou, Zhejiang 325027 (CN); ZHANG, Sen, Wenzhou, Zhejiang 325027 (CN); QU, Jia, Wenzhou, Zhejiang 325027 (CN); ZHENG, Qinyuan, Wenzhou, Zhejiang 325027 (CN)
(74) Representative: Huang, Liwei
(86) International application number: PCT/CN2022/106704
(87) International publication number: WO 2023/001176

(57) **Abstract**

The present application relates to a method for treating myopia with vipocetine, which can effectively prevent myopia and/or control the progression of myopia while being safe and free of obvious side effects, having good prospects for clinical application.

## Description

### Technical Field

The present application relates to a method of treating myopia with vinpocetine, in particular to a method and use for controlling the progression of myopia, and provides a corresponding drug or preparation, and belongs to the field of medicine.

### Background

Myopia is the most common refractive error. Refractive error refers to a refractive state in which the focus falls in front of the retina after the parallel light is refracted by the refractive system of the eye in a relaxed state of accommodation. Myopia causes blurry vision of distant objects, which brings many inconveniences to life. Although the distance vision of myopia patients can be improved through correction means such as framed glasses, such technologies or methods cannot improve the social phenomenon of high myopia rate and increasing myopia degree. Therefore, the number of people who have myopia worldwide is increasing year by year, especially in East Asian countries, the situation of patients with high myopia is worrying. However, there are many types of myopia and the pathogenesis is still unclear. There is currently a lack of effective and safe drugs to treat myopia, especially to control the progression of myopia. Therefore, there is an unmet clinical need for this disease.

Vinpocetine has a chemical formula of C₂₂H₂₆N₂O₂, has an average molecular weight of 350.46 and is a white crystalline powder, soluble in chloroform or 96% ethanol, almost insoluble in water, derived from an indole alkaloid extracted from *Vinca minor* of the *Apocynaceae* family, and has a structure as follows:

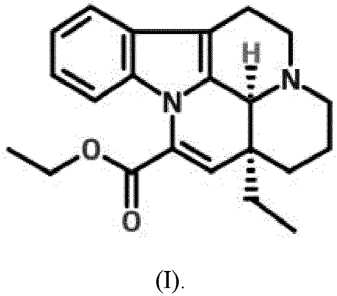

Vinpocetine's solubility in water is pH-dependent, and vinpocetine is found to be more soluble in gastric environment pH (1.2) and intestinal environment pH (6.8), and has higher biological activity and less toxic side effects than vincamine. Vinpocetine has high lipid solubility. This compound can enter the blood-brain barrier, blood-eye barrier, placenta and breast milk, and has high pharmacological activity on cardiovascular and cerebrovascular and central nervous system functions.

Currently, vinpocetine is mainly used for the treatment of cerebrovascular-related diseases and cognitive impairment, and most of the recipients are the elderly. It is reported that after systemic administration, vinpocetine can selectively act on the cerebrovascular system, inhibit phosphodiesterase activity in the brain (*i.e*., act as a phosphodiesterase inhibitor), relax vascular smooth muscle, and increase blood supply to the brain. This type of drugs has the characteristics of rapid metabolism in the body, short elimination half-life, and low human bioavailability in oral dosage forms. Since vinpocetine for external use in the eye can easily cross physical barriers such as the cornea and lens, vinpocetine is also an auxiliary ingredient in many ophthalmic medications and is mainly used as a microcirculation promoter to promote eye blood flow. Although promoting blood circulation in the eyes may make the eyes feel more comfortable, thereby increasing the visual acuity of patients with refractive errors, this is not equivalent to myopia treatment, and there are even reports that the administration of vinpocetine may actually cause myopia (biosintez.com/en/catalog/product/283).

### Summary of the Invention

This application unexpectedly discovered a novel use of vinpocetine and proved through repeated trials that: vinpocetine can treat myopia and control myopia progression. That is, vinpocetine is not used as a supplement or auxiliary ingredient, such as a microcirculation promoter (inhibiting phosphodiesterase activity), to assist, accompany or help other active substances used for treating myopia in the medicine for treating myopia to enhance (for example, make more main ingredients of the medicine effectively reach target cells) the effect of the active substances for treating myopia; instead, vinpocetine serves as a direct active ingredient in the treatment of myopia.

This application also studies the mechanism of vinpocetine in treating myopia. Before this application, people used vinpocetine as a phosphodiesterase inhibitor to enhance blood flow. However, this application found through experiments that vinpocetine does not rely on choroidal blood flow improvement when treating myopia and controlling myopia progression. In other words, vinpocetine does not treat myopia and inhibit myopia progression by dilating blood vessels or promoting blood circulation (*i*.*e*., the known functions of phosphodiesterase inhibitors), but may rely more on an inherent but still unknown new pharmacological effects. Therefore, this application actually exploits an unknown function of vinpocetine, and this discovery also forms the key basis of this application.

This application proves for the first time that vinpocetine alone can have a significant inhibitory effect on the progression of myopia, and thus confirms that vinpocetine can effectively treat various types of myopia including simple myopia or axial myopia (such as juvenile myopia), mild or moderate myopia and the like. Compared with using vinpocetine to treat myopia in the elderly, or high myopia, or high myopia in the elderly, or pathological myopia, vinpocetine has a significantly better effect in the treatment of myopia with declining refraction (such as simple myopia, axial myopia, or axial simple myopia, or low and moderate myopia, or myopia of children and adolescents, or progressive myopia and the like).

Vinpocetine can also be used to prevent various types of myopia. For example, using vinpocetine for drug intervention in the progression stage of myopia can prevent and stop the occurrence of myopia in the elderly, high myopia, high myopia in the elderly and pathological myopia.

This application further studies the concentration, ratio and formula of vinpocetine in drugs or preparations for treating myopia, as well as the administration frequency of related drugs, combined treatment and other medication methods. Among them, local (eye) administration is not only safe but also has a significantly better therapeutic effect than systemic administration.

This application provides use of vinpocetine, or an optical isomer or a racemate, or a solvate, or a pharmaceutically acceptable salt, or a prodrug, or a metabolite, or a related compound or extract, or a crystalline compound thereof, or a combination of these substances, the use is selected from one of the following requirements or two or more of the following requirements:
① preventing and/or treating myopia and related symptoms thereof, and/or myopia correction; or
② inhibiting (controlling) the progression of myopia; or
③ preventing and controlling or delaying the occurrence and development of myopia (myopic eye); or
④ inhibiting and slowing down axial length elongation and/or vitreous chamber length (depth) increase in an individual with myopia or an individual with a tendency to develop myopia; or
⑤ preventing , slowing down, attenuating or treating the abnormal development of eyeballs associated with visual impairment; or
⑥ allowing an individual to obtain clearer distance vision without wearing glasses (such as framed glasses or OK lenses) or relying on vision correction means (such as refractive surgery) than the distance vision before using these substances or means and/or than the distance vision without using these substances or means; or
⑦ inhibiting, delaying or slowing down the procession or speed of the refraction change towards negative in an individual with myopia or an individual with a tendency to develop myopia; or
⑧ preparing a drug, a preparation, a composition or a device, which are used to achieve at least one of ①-⑦ as stated above.

In some embodiments, systemic administration (such as oral administration, intravenous drip), and/or local administration (eye drops, eye injection, eye implant, skin ointment/periorbital emulsion application or eye ointment application), and/or parenteral administration (such as mucosal administration, transdermal administration, microneedle administration), and/or non-invasive administration (such as administration via eye spray) are adopted.

In some embodiments, the drug, preparation or composition can be an injection, a tablet, a freeze-dried powder for injection, a capsule, an effervescent tablet, a chewable tablet, a buccal tablet, a granule, an ointment, a syrup, an oral liquid, an aerosol, a nasal drop, a topical preparation, an oral preparation, *etc*.; preferred is an ocular dosage form, including but not limited to an eye drop (eyewash), an eye ointment, an eye spray, an implant tablet, an eye gel, an eye patch, an eye microsphere, an eye sustained-release preparation, a periorbital injection, and an intraocular injection; the drug, preparation or composition can also be a free solution, an oil-water mixture, a suspension, a liniment, a lotion, a cream, a drop, a electuary, a spray, an ointment, a patch, a paste, a pill, a suppository and an emulsion, and can contain celluloses (such as methylcellulose), polyhydric alcohols, cyclodextrins (such as hydroxypropyl-β-cyclodextrin), acidic co-solvents (such as citric acid), dendrimers, nanomaterials, sustained-release materials, liposomes or a combination formulation group of these substances.

In some embodiments, the individual with myopia or the individual with a tendency to develop myopia is a child and/or an adolescent, preferably a person aged 3 to 26 years old, more preferably a person aged 6 to 18 years old; or a juvenile, preferably a person whose eyes (eyeballs) are still at the growth and development stages; or school-age people, preferably a student in grades 1 to 12; or people whose parents are highly myopic; or people with insufficient reserves for distance vision.

In some embodiments, the myopia is refractive myopia or axial myopia; congenital myopia (myopia is present at birth or before school age), early-onset myopia (under 14 years old), delayed onset myopia (16 to 18 years old), or late-onset myopia (after adulthood); low myopia (mild myopia), moderate myopia, high myopia (severe myopia); pseudomyopia or true myopia; myopia in children and/or adolescents (preferably, aged 3-26 years old, more preferably, aged 6-18 years old), myopia in juveniles, myopia in adults, or myopia in the elderly; simple myopia or pathological myopia; axial simple myopia or simple axial myopia; axial myopia in children and/or adolescents (preferably, aged 3-26 years old, more preferably, aged 6-18 years old); axial myopia in school-age and preschool-age people; primary myopia or secondary myopia; primary myopia in children and/or adolescents (preferably, aged 3-26 years old, more preferably, aged 6-18 years old); or progressive myopia in children and/or adolescents (preferably, aged 3-26 years old, more preferably, aged 6-18 years old); curvature myopia, index myopia, astigmatic myopia, position myopia or bending myopia; axial myopia in which the refraction continuously changes towards negative; myopia caused by long-term use of eyes at close range, myopia and pseudomyopia caused by visual fatigue, refraction change towards negative caused by adverse drug reactions, myopic eye, myopia caused by reading books, myopia caused by using electronic products such as mobile phones, myopia caused by mismatch of refractive media (components), refractive myopia, myopia caused by abnormal refractive development, myopia caused by excessive eyeball growth, myopia caused by unhygienic use of eyes, the imaging focus of distant objects falling in front of the retina due to various reasons, myopia that is poorly or ineffectively treated with atropine, myopia caused by insufficient outdoor exercise, accommodative spastic myopia, myopia in children, myopia in infants, hereditary myopia, and myopia dominated by environmental factors.

In some embodiments, the related symptoms of myopia include complications caused by myopia, such as complications of high myopia, and for example, floaters, glaucoma, posterior scleral staphyloma, retinal detachment, retinal tear, amblyopia, macular hemorrhage, choroidal neovascularization, choroidal atrophy, macular degeneration or maculopathy, visual field defect, progressive or sudden decrease in vision (especially near vision), eye soreness and/or pain, night blindness, astigmatism, anisometropia, blindness, vitreous liquefaction, vitreous opacity, squint, frequent blinking, frequent eye rubbing, anisometropia, blurred vision when looking at distant objects, need to squint or partially close eyelids to see distant objects clearly, headache caused by eye strain, inattention caused by myopia, difficulty seeing while driving, especially at night (nocturnal myopia), retinal atrophy and degeneration (hemorrhages and holes), subretinal neovascularization, or eyeball atrophy.

In some embodiments, the drug, preparation, composition or device further comprises other drugs or ophthalmic preparations, including but not limited to drugs for myopia treatment (s such as pirenzepine, muscarinic antagonist, robaxin, indoramine, timolol maleate, epinephrine, pyrazine, perlapine, methylamine, chlorisondamine, acetylcholinesterase inhibitor, dopamine agonist, gamma-aminobutyric acid, naloxone, glucagon, retinoic acid, salidroside, formononetin, etc.), M receptor blockers (such as blockers or antagonists or inhibitors targeting M2 or M3 receptors), bendazac and various salt forms thereof (bendazac lysine), atropine, dibazole, polyunsaturated fatty acids (such as DHA, EPA), prazosin, homatropine, anisodamine (racemic), tropicamide, 7-methylxanthine, niacin, piracetam, Salvia miltiorrhiza extract, safflower extract, fish oil, bear bile extract, vitamins, adenosine triphosphate (ATP), smooth muscle relaxants, drugs to prevent vasospasm, non-selective adenylate antagonist, vasodilators, pupil dilating components, decongestants, eye muscle (e.g., ciliary muscle) accommodative components, anti-inflammatory (antiphlogistic) agent components, astringent components, antihistamine components, anti-allergic components, components for inhibiting collagen degradation, hepatoprotective (avoiding or reducing liver toxicity) components, components for enhancing blood-retinal barrier (making it more difficult for compounds to penetrate this physiological barrier), amino acids, antibacterial agent components, antioxidant components, saccharides, polymers or derivatives thereof, celluloses or derivatives thereof, local anesthetic components, components for amblyopia treatment, components for glaucoma treatment, components for cataract treatment, type I phosphodiesterase inhibitors (specific or non-specific), miRNA and modified derivatives thereof, therapeutic components for ophthalmic diseases, excipients, *etc.*

In some embodiments, vinpocetine, or the optical isomer or the racemate, or the solvate, or the pharmaceutically acceptable salt, or the prodrug, or the metabolite, or the related compound or extract, or the crystalline compound thereof, or the combination of these substances, and one or more drugs for prevention, control and/or treatment of myopia are formulated or designed to be administered continuously, simultaneously, or sequentially, or alternately, or at intervals, or separately.

In some embodiments, the related compound or extract of vinpocetine is selected from one or more of vinpocetine metabolites (such as apovincaminic acid, hydroxyvinpocetine, hydroxyl-apovincaminic acid and dihydroxy-vinpocetine-glycinate), vinca alkaloid, apovincamine, vinca extract (periwinkle), vincamine, voacanga africana extract, Apocynaceae plant extract, vincristine, tabersonine, vinblastine, vindoline, apovincaminic acid hydrochloride, catharanthine, ethyl vincaminate, methoxyvinpocetine and dihydrovinpocetine.

In some embodiments, the preparation is a health care product, a food, a dietary supplement, a nutritional product, a drink and other oral products or cosmetics. The cosmetics can be a combination of one or more of a free solution, an oil-water mixture, a suspension, a liniment, a lotion, a spray, a cream, a drop, an electuary, an ointment, a paste, a pill, a suppository, an emulsion and a patch.

In some embodiments, the device is an instrument, an equipment, a consumable, a system, a medical device, a health care product or an article for changing the appearance of eyes, such as a corneal contact lens, a glass, an intraocular lens, a suture line, an OK lens cleaning (maintenance) system, an eye patch, a patch for improving eyesight, a coloured contact lens, a microneedle, an eye spray system, an eye massager, an eye fumigation device, an ocular surface drug delivery device, an intraocular drug delivery device, a fundus drug delivery device, an implantable pump, a wearable device, an acupoint massager, an eye relaxation device, a myopia treatment device or a drug-device combination for myopia prevention and control.

In some embodiments, vinpocetine, or the optical isomer or the racemate, or the solvate, or the pharmaceutically acceptable salt, or the prodrug, or the metabolite, or the related compound or extract, or the crystalline compound thereof, or the combination of these substances is used as a sole active ingredient or a main active ingredient or a direct active ingredient; or the content or efficacy of vinpocetine, or the optical isomer or the racemate, or the solvate, or the pharmaceutically acceptable salt, or the prodrug, or the metabolite, or the related compound or extract, or the crystalline compound thereof, or the combination of these substances accounts for 1% or less, or more than 1%, more than 10%, more than 20%, more than 30%, more than 40%, more than 50%, more than 60%, more than 70%, more than 80%, more than 90% or 100% of all active ingredients in the use, and the percentage (%) can be a mass ratio, a molar ratio, or a titer ratio.

In some embodiments, the concentration of vinpocetine, or the optical isomer or the racemate, or the solvate, or the pharmaceutically acceptable salt, or the prodrug, or the metabolite, or the related compound or extract, or the crystalline compound thereof, or the combination of these substances in the drug, preparation, composition or device is 0.001 µM to 10 mM, preferably 0.01 µM to 1000 µM, preferably 0.05 µM to 100 µM, more preferably 0.1 µM to 25 µM, more preferably 0.1 µM to 15 µM; or the concentration or proportion of these substances or their combination in the drug, preparation, composition or device is less than 25%, preferably less than 5%, preferably less than 1%, more preferably less than 0.01%, more preferably less than 0.001%, and the percentage (%) can be mass/volume concentration (g/100 ml) or mass percentage or mole (number) ratio.

The present application provides a drug, preparation, composition or device for ocular administration, the concentration of vinpocetine, or the optical isomer or the racemate, or the solvate, or the pharmaceutically acceptable salt, or the prodrug, or the metabolite, or the related compound or extract, or the crystalline compound thereof, or the combination of these substances in the drug, preparation, composition or device is 0.001 µM to 10 mM, preferably 0.01 µM to 1000 µM, preferably 0.05 µM to 100 µM, more preferably 0.1 µM to 25 µM, more preferably 0.1 µM to 15 µM; or the concentration or proportion of these substances or their combination in the drug, preparation, composition or device is less than 25%, preferably less than 5%, preferably less than 1%, more preferably less than 0.01%, more preferably less than 0.001%, and the percentage (%) can be mass/volume concentration (g/100 ml) or mass percentage or mole (number) ratio.

The present application further provides a pharmaceutical composition or compound preparation containing at least 2 active substances, comprising vinpocetine, or an optical isomer or a racemate, or a solvate, or a pharmaceutically acceptable salt, or a prodrug, or a metabolite, or a related compound or extract, or a crystalline compound thereof, or a combination of these substances, and other active substances for treating myopia. Preferably, the added amount, concentration and/or efficacy of vinpocetine, or the optical isomer or the racemate, or the solvate, or the pharmaceutically acceptable salt, or the prodrug, or the metabolite, or the related compound or extract, or the crystalline compound thereof, or the combination of these substances is not lower than the added amount, concentration and/or efficacy of any other active substances for treating myopia, and the efficacy is aimed at treating myopia or controlling myopia progress.

The present application further provides a pharmaceutical composition or compound preparation, comprising vinpocetine, or an optical isomer or a racemate, or a solvate, or a pharmaceutically acceptable salt, or a prodrug, or a metabolite, or a related compound or extract, or a crystalline compound thereof, or a combination of these substances, and other active substances for treating myopia, and the other active substances for treating myopia do not comprise long-acting beta adrenergic receptor agonists, long-acting muscarinic antagonists and/or muscarinic antagonists.

The present application further provides an eye preparation or drug, in which vinpocetine, or the optical isomer or the racemate, or the solvate, or the pharmaceutically acceptable salt, or the prodrug, or the metabolite, or the related compound or extract, or the crystalline compound thereof, or the combination of these substances is a direct, sole or main active ingredient.

In some embodiments, the preparation or drug includes, but not limited to, an ocular dosage form, including but not limited to an eye drop (eyewash), an eye ointment, an eye spray, an implant tablet, an eye gel, an eye patch, an eye microsphere, an eye sustained-release preparation, a periorbital injection, or an intraocular injection.

In some embodiments, the concentration of vinpocetine, or the optical isomer or the racemate, or the solvate, or the pharmaceutically acceptable salt, or the prodrug, or the metabolite, or the related compound or extract, or the crystalline compound thereof, or the combination of these substances is 0.001 µM to 10 mM, preferably 0.01 µM to 1000 µM, preferably 0.05 µM to 100 µM , more preferably 0.1 µM to 25 µM, more preferably 0.1 µM to 15 µM; or the concentration or proportion of vinpocetine is less than 25%, preferably less than 5%, preferably less than 1%, more preferably less than 0.01%, more preferably less than 0.001%, and the percentage (%) can be mass/volume concentration (g/100 ml) or mass percentage or mole (number) ratio.

In some embodiments, the type I phosphodiesterase inhibitor is PDE1-IN-2, LY1 (Sherif Khedr et al., Selective Phosphodiesterase 1 Inhibitor LY1 Reduces Blood Pressure in Spontaneously Hypertensive and Dahl Salt Sensitive Rats, The FASEB Journal, 2020 April 1, Volume34, IssueS1, Pages 1-1.), Lu AF41228, Lu AF58027 (Morten Laursen et al., Novel selective PDE type 1 inhibitors cause vasodilatation and lower blood pressure in rats, Br J Pharmacol, 2017 Aug;174(15):2563-2575.), BTTQ (Asim B Dey et al., elective Phosphodiesterase 1 Inhibitor BTTQ Reduces Blood Pressure in Spontaneously Hypertensive and Dahl Salt Sensitive Rats: Role of Peripheral Vasodilation, Front Physiol, 2020 Sep 8; 11:543727.), Nimodipine, Zaprinast, Caffeine, Deprenyl, Amantadine(Alexandre E Medina, Therapeutic utility of phosphodiesterase type I inhibitors in neurological conditions, Front Neurosci,. 2011 Feb 18; 5:21.), KS505a, bepril, flunarizine, amiodarone, zaprinast, 8-methoxymethyl IPMX, SCH 51866, Nimodipine, IC224(Claire Lugnier,Cyclic nucleotide phosphodiesterase (PDE) superfamily: a new target for the development of specific therapeutic agents, Pharmacol Ther, 2006 Mar;109(3):366-98.), ITI-214(Peng Li et al., Discovery of Potent and Selective Inhibitors of Phosphodiesterase 1 for the Treatment of Cognitive Impairment Associated with Neurodegenerative and Neuropsychiatric Diseases, J Med Chem, 2016 Feb 11;59(3): 1149-64.), *etc.* Since vinpocetine is a type I phosphodiesterase inhibitor (PDE1 inhibitor), perhaps other type I phosphodiesterase inhibitors known before the filing date (whether specific or not) have the same unknown activity or efficacy as vinpocetine, such as the ability to prevent and/or treat myopia and control myopia progression.

In some embodiments, the miRNA and modified derivatives thereof are MicroRNA-328 or MicroRNA-328 anti-strand compositions.

In some embodiments, two or more substances selected from vinpocetine, or the optical isomer or the racemate, or the solvate, or the pharmaceutically acceptable salt, or the prodrug, or the metabolite, or the related compound or extract, or the crystalline compound thereof are combined and administered in the same time period, such as simultaneously or sequentially during a specific administration (treatment), on the same day, in the same week, or in the same month, or in the same year; or alternatively at intervals, such as alternating administration at 4-hour intervals, alternating administration at 12-hour intervals, alternating administration on every other day, alternating administration every other week, alternating administration every other month, and alternating administration every other year; or:
vinpocetine, or the optical isomer or the racemate, or the solvate, or the pharmaceutically acceptable salt, or the prodrug, or the metabolite, or the related compound or extract, or the crystalline compound thereof, or the combination of these substances and one or more other drugs are administered in the same time period, such as simultaneously or sequentially during a specific administration (treatment), on the same day, in the same week, or in the same month, or in the same year; or alternatively at intervals, such as alternating administration at 4-hour intervals, alternating administration at 12-hour intervals, alternating administration on every other day, alternating administration every other week, alternating administration every other month, and alternating administration every other year; or:
vinpocetine, or the optical isomer or the racemate, or the solvate, or the pharmaceutically acceptable salt, or the prodrug, or the metabolite, or the related compound or extract, or the crystalline compound thereof, or the combination of these substances are used in combination with devices (such as corneal contact lenses) and/or surgeries (such as refractive correction surgery, corneal laser surgery for myopia, and lens surgery).

In some embodiments, the one or more other drugs are myopia prevention and control and/or myopia treatment drugs (such as bendazac lysine, atropine, dibazole, polyunsaturated fatty acids, DHA, fish oil, prazosin, M receptor blockers, niacin, pirenzepine, muscarinic antagonist, 7-Methylxanthine (7MX), pirenzepine, muscarinic antagonist, Robaxin, indoramine, timolol maleate, epinephrine, pyrazine, perlapine, methylamine, chlorisondamine, adrenaline, acetylcholinesterase inhibitor, dopamine agonist, gamma-aminobutyric acid, naloxone, glucagon, retinoic acid, salidroside, formononetin, etc.), vasodilators, smooth muscle relaxants, drugs to prevent vasospasm, drugs to regulate collagen metabolism, piracetam, antiallergic drugs, hepatoprotective drugs, vinpocetine, or an optical isomer or a racemate, or a solvate, or a pharmaceutically acceptable salt, or a prodrug, or a metabolite, or a related compound or extract, or a crystalline compound thereof, or a combination of these substances and the like.

In some embodiments, the device is an instrument, an equipment, a consumable, a medical device, or a health care product that has the function of protecting vision or treating (correcting) myopia, such as various glasses, OK lenses, framed glasses, eye patches, (myopia) acupoint massagers, eye relaxation devices, and myopia treatment devices that delay myopia progression.

In some embodiments, the drug, one or more other drugs, myopia prevention and control drugs, myopia treatment drugs, or ophthalmic preparations may not be long-acting β-adrenoceptor agonists (LABAs), long-acting muscarinic antagonists (LAMAs) and/or muscarinic antagonists, for example, may not be salmeterol and/or tiotropium.

In some embodiments, in the process of achieving the described uses, systemic dosage forms (such as oral tablets) and local dosage forms (such as eye drops) are used at the same time, or in combination, or alternately, or at intervals, or alone.

In some embodiments, the abnormal development of the eyeball is an abnormal refractive development mainly induced by environmental factors or mainly caused by human factors (such as long-term reading at close range, frequent use of electronic screens, continuous near vision and lack of far vision opportunities, improper use of corrective glasses, side effects of drugs, obesity, trauma, poor lighting in the learning environment, and lack of outdoor exercises) which has nothing to do with genetic factors, or genetic factors are secondary factors, concomitant factors, or synergistic factors. The abnormal development is, for example, abnormal development of the size of the eyeballs in children and adolescents (e.g., 3-26 years old).

In some embodiments, abnormal development or developmental abnormalities include an excessively long length or a mismatch between the axial length elongation speed and the refractive system, causing the imaging focus of parallel light rays to be located in front of the retina after passing through the normal or abnormal refractive system of the eye.

### Brief Description of the Figures

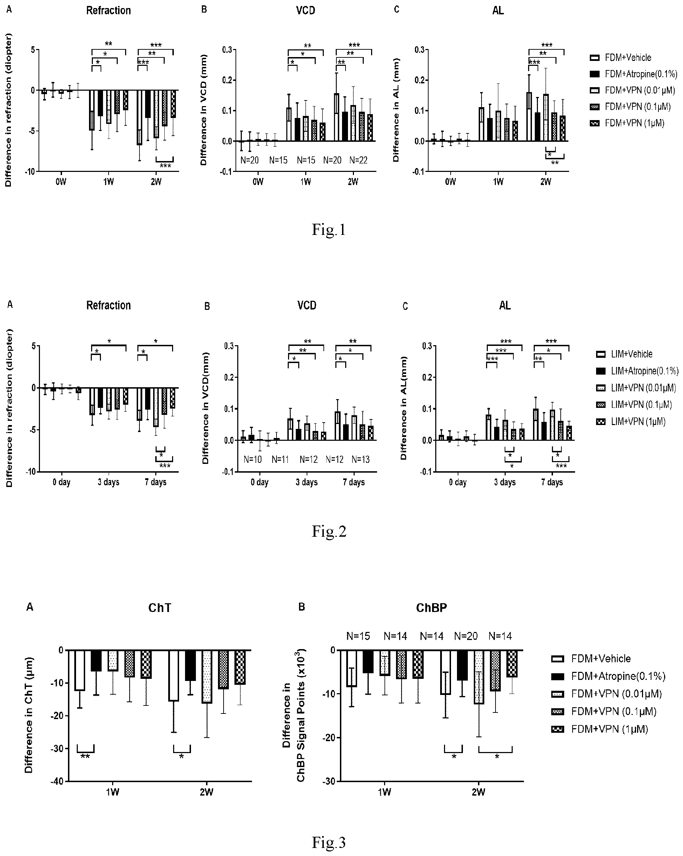

Fig. 1: Effects of 3 concentrations, that is, high, medium and low concentrations of vinpocetine on refraction (degree), vitreous chamber depth and axial length in the FDM group. Among them, A is the diagram showing refraction difference between the experimental eye and the contralateral eye; B is the diagram showing vitreous chamber depth difference between the experimental eye and the contralateral eye; C is the diagram showing axial length difference between the experimental eye and the contralateral eye.
Fig. 2: Effects of 3 concentrations, that is, high, medium and low concentrations of vinpocetine on refraction (degree), vitreous chamber depth and axial length in the LIM group. Among them, A is the diagram showing refraction difference between the experimental eye and the contralateral eye; B is the diagram showing vitreous chamber depth difference between the experimental eye and the contralateral eye; C is the diagram showing axial length difference between the experimental eye and the contralateral eye.
Fig. 3: Effects of 3 concentrations, that is, high, medium and low concentrations of vinpocetine on choroidal thickness and blood flow in the FDM group. Among them, A is the effect of vinpocetine on choroidal thickness ChT; B is the effect of vinpocetine on choroidal blood flow ChBP.

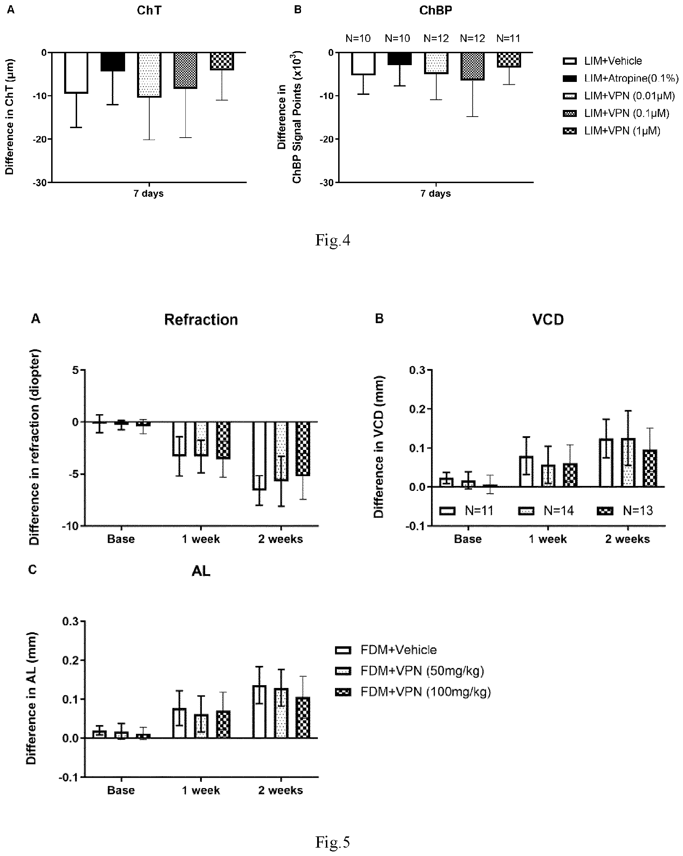

Fig. 4: Effects of 3 concentrations, that is, high, medium and low concentrations of vinpocetine on choroidal thickness and blood flow in the LIM group. Among them, A is the effect of vinpocetine on choroidal thickness ChT; B is the effect of vinpocetine on choroidal blood flow ChBP.

Notes: * represents p < 0.05, ** represents p < 0.01, *** represents p < 0.001.

In each different time period (LIM group: before administration, 3 days of administration and 7 days of administration, or FDM group: before administration, 1 week of administration and 2 weeks of administration), 5 bars are used to show the determination results. In Fig. 1 and Fig. 3, every 5 consecutive bars from left to right are FDM+Vehicle, FDM+Atropine(0.1%), FDM+VPN(0.01 µM), FDM+VPN(0.1 µM), and FDM+ VPN(1 µM). In Fig. 2 and Fig. 4, every 5 consecutive bars from left to right are LIM+Vehicle, LIM+Atropine (0.1%), LIM+VPN (0.01 µM), LIM+VPN (0.1 µM), and LIM+ VPN(1 µM). VPN = vinpocetine.

Fig. 5: Changes in refraction and axial parameters of myopic individuals under systemic administration of vinpocetine at different doses. Compare the differences in refraction (A), vitreous chamber depth (B) and axial length (C) between the eyes of subject individuals before the experiment, by 1 week of the experiment, and by 2 weeks of the experiment. VPN: vinpocetine; Vehicle: solvent; Week: week; Refraction: refraction; VCD: vitreous chamber depth; AL: axial length; N: sample size. Variance analysis of repeated measurements (A-C).

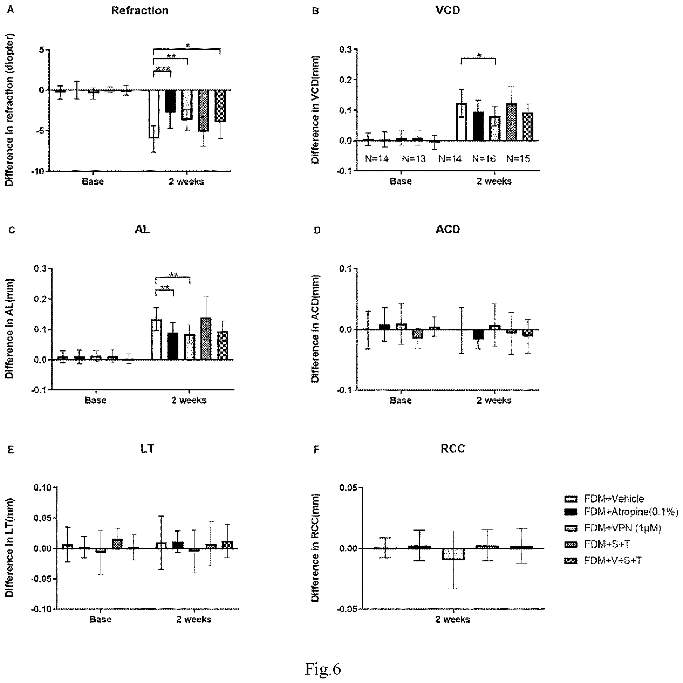

Fig. 6: Treatment of myopia with salmeterol and tiotropium. Compare the effect of change in the difference in refraction (A), vitreous chamber depth (B), axial length (C), anterior chamber depth (D), lens thickness (E) and corneal curvature (F) between the eyes of myopic individuals in different drug groups before and after the experiment. VPN = V: vinpocetine; T: tiotropium; S: salmeterol; Vehicle: solvent; Week: week; Refraction: refraction; VCD: vitreous chamber depth; AL: axial length; ACD: anterior chamber depth; LT: crystal thickness; RCC: corneal curvature; N: sample size. * P < 0.05, **P < 0.01, ***P < 0.001. Variance analysis of repeated measurements (A-F).

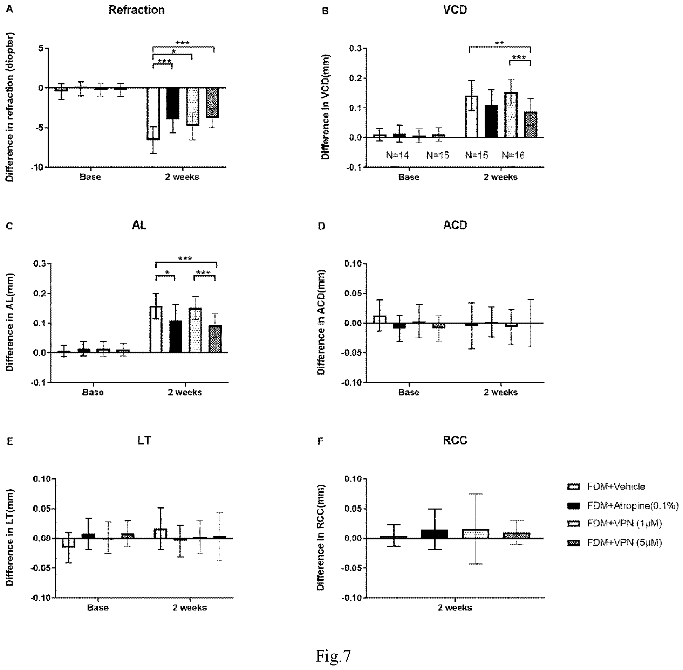

Fig. 7: The intervention effect of vinpocetine eye drops on the refraction, vitreous chamber depth and axial length of myopic individuals. Compare the change in the difference in refraction (A), vitreous chamber depth (B), axial length (C), anterior chamber depth (D), lens thickness (E) and corneal curvature (F) between the eyes of the same subject individual in different drug groups before experiment and after 2 weeks of administration. VPN: vinpocetine; Vehicle: solvent; Refraction: refraction; VCD: vitreous chamber depth; AL: axial length; ACD: anterior chamber depth; LT: crystal thickness; RCC: corneal curvature; N: sample size. * P < 0.05, **P < 0.01, ***P < 0.001. Variance analysis of repeated measurements (A-F).

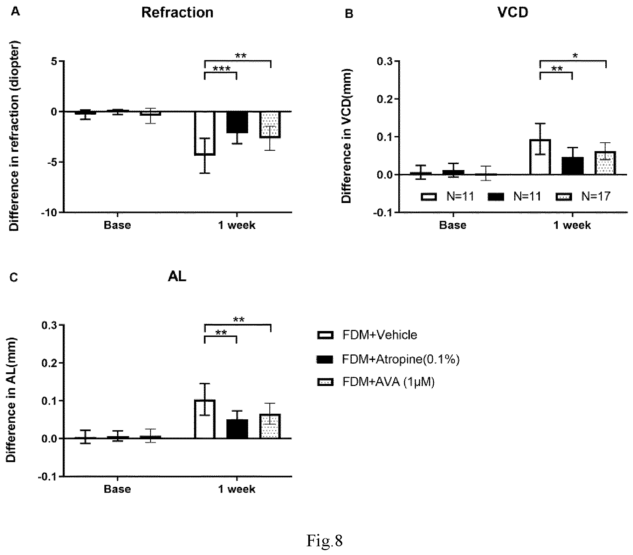

Fig. 8: The intervention effect of vinpocetine metabolite AVA on the refraction, vitreous chamber depth and axial length of myopic individuals. Compare the differences in refraction (A), vitreous chamber depth (B) and axial length (C) between the eyes of subject individuals before the experiment and by 1 week of the experiment. AVA: apovincaminic acid, a metabolite of vinpocetine; Vehicle: solvent; Week: week; Refraction: refraction; VCD: vitreous chamber depth; AL: axial length; N: sample size. * P < 0.05, **P < 0.01, ***P < 0.001. Variance analysis of repeated measurements (A-C).

### Detailed Description of Embodiments

Embodiments of the present application will be exemplarily shown below. Although the present application has been described in connection with these specific embodiments, it should be understood that there is no intention to limit the application to such specific embodiments. On the contrary, the application is intended to cover alternatives, modifications, substitutions, variations and equivalents as may be included within the spirit and scope of the application as defined by the appended claims.

The present application provides a method for preventing and treating myopia, and the method comprises the local administration of vinpocetine or a related compound thereof to the eye.

The present application further provides a drug for myopia treatment and myopia progression control that is locally administrated to the eye and uses vinpocetine as the sole or main active ingredient. In the prior art, vinpocetine is mainly available as tablets and injections, and is administered systemically. When vinpocetine is used to treat myopia, if vinpocetine is administered systemically, it would be affected by the first-pass effect of the drug and the blood-retinal barrier (BRB) and it is necessary to give a large dose of the drug to ensure that vinpocetine or a metabolite thereof entering the eye reach the therapeutic concentration threshold. Such a treatment strategy has the potential risk of systemic adverse reactions, especially for people who is young and requires long-term continuous administration. At the same time, systemic administration is affected by differences of more individuals, which increases the uncertainty of the efficacy of vinpocetine in preventing and controlling myopia. Therefore, the present application developed a vinpocetine drug with better efficacy and safety for local administration in the eye. This drug development strategy is aimed at the use of vinpocetine and a related compound thereof (such as fluoride) in the prevention and control of myopia in children and adolescents. The advantage is that patients have convenient medication, high compliance, and can be administered for a long time (such as one month, six months, one year, three years, five years and ten years), the growth and development of young individuals is less affected, and the ocular dosage form is safe and reliable.

The present application also provides a method for administering an effective dose of vinpocetine or a related compound thereof to a suitable subject for treating myopia and/or inhibiting axial length elongation thereof.

The method or pharmaceutical composition of the present application can significantly inhibit myopia, especially myopia in children and/or adolescents (preferably the age group is 3-26 years old, more preferably the age group is 6-18 years old), or early and middle-stage myopia, or mild or moderate myopia, or non-pathological myopia, or axial simple myopia, or axial myopia in children and/or adolescents (preferably the age group is 3-26 years old, more preferably the age group is 6-18 years old), or progressive myopia in children and/or adolescents (preferably the age group is 3-26 years old, more preferably the age group is 6-18 years old), or primary myopia, or non-senile myopia, or juvenile myopia, or progressive myopia, or non-refractive myopia.

The method or pharmaceutical composition of the present application can significantly inhibit and slow down the axial length elongation and/or the vitreous chamber length increase in individuals with myopia (including individuals who are not yet myopic but will develop myopia). Preferably, the method or pharmaceutical composition of the present application is applicable to the population including those who have myopia in children and/or adolescents (preferably the age group is 3-26 years old, more preferably the age group is 6-18 years old), or early and middle-stage myopia, or mild or moderate myopia, or non-pathological myopia, or axial simple myopia, or axial myopia in children and/or adolescents (preferably the age group is 3-26 years old, more preferably the age group is 6-18 years old), or progressive myopia in children and/or adolescents (preferably the age group is 3-26 years old, more preferably the age group is 6-18 years old), or primary myopia, or non-senile myopia, or juvenile myopia, or progressive myopia, or non-refractive myopia, these myopic individuals (including individuals who are not yet myopic but will develop myopia) have their axial length elongation and/or the vitreous chamber length increase inhibited or even terminated by the method or pharmaceutical composition of the present application.

The present application provides a method for treating, preventing or slowing down myopia and related symptoms in a subject, comprising administering to the subject a therapeutically effective amount of vinpocetine or a therapeutically acceptable salt thereof or a related compound thereof. Preferably, the vinpocetine is administered alone. Preferably, the vinpocetine is administered with other drugs simultaneously, sequentially or at intervals, or alternately. Preferably, the vinpocetine is administered in the form of a pharmaceutical composition. Preferably, the pharmaceutical composition is prepared into an ocular preparation. Preferably, the ocular preparation further contains a pharmaceutically acceptable carrier. Preferably, the carrier is an ophthalmologically acceptable carrier.

The present application also provides a pharmaceutical composition for treating, preventing or slowing down myopia and related symptoms, the pharmaceutical composition comprises vinpocetine or a therapeutically acceptable salt thereof or a related compound thereof. Preferably, the pharmaceutical composition is prepared into an ocular preparation. Preferably, the ocular preparation further contains a pharmaceutically acceptable carrier. Preferably, the carrier is an ophthalmologically acceptable carrier.

This application also provides the use of a compound or a therapeutically acceptable salt thereof and a derivative thereof in the preparation of a pharmaceutical composition for treating, preventing or slowing down myopia and related symptoms thereof. The compound may be a vinpocetine metabolite (such as apovincaminic acid, Vinca alkaloid, apovincamine, Vinca extract (periwinkle), vincamine, African voacanga extract (voacanga africana), Apocynaceae plant extract, vincristine, tabersonine, Vinblastine, Vindoline, Apovincaminic Acid Hydrochloride, catharanthine, ethylvincaminate, *etc.* Preferably, the compound is vinpocetine or a therapeutically acceptable salt thereof and a derivative thereof. Preferably, the pharmaceutical composition is prepared into an ocular preparation. Preferably, the ocular preparation further contains a pharmaceutically acceptable carrier. Preferably, the carrier is an ophthalmologically acceptable carrier.

The related symptoms of myopia include but are not limited to: floaters, glaucoma, posterior scleral staphyloma, retinal detachment, amblyopia, macular hemorrhage, visual field defects, progressive or sudden decrease in vision (especially near vision), eye soreness and/or pain and night blindness.

According to one aspect of the present application, the myopia is refractive myopia. According to another aspect of the application, the myopia is axial myopia.

This application also provides a myopia treatment (inhibition of myopia progression) scheme in which vinpocetine or a salt form thereof is used in combination with or at intervals or alternately with other myopia prevention and control drugs (such as atropine) or optical prevention and control means (such as OK lenses) in the process of preventing, slowing down and treating myopia.

This application also provides a myopia treatment (inhibition of myopia progression and/or axial length elongation) scheme in which vinpocetine or a salt form thereof is used in combination or alternately with systemic dosage forms (such as oral tablets) and topical dosage forms (such as eye drops) in the process of preventing, slowing down and treating myopia.

### Terms and definitions

"Myopia" means that in a relaxed state of accommodation, parallel light rays gather in front of the retina after passing through the refractive system of the eyeball. Myopia is usually characterized by far vision lower than normal level and normal near vision. The clinical concept of myopic individuals is: those with static refraction ≥ -0.25D. The clinical manifestations are blurry long-distance vision and good near-distance vision. In the early stage of myopia, long-distance vision often fluctuates. Since no or less accommodation is needed for near vision, the convergence function is correspondingly weakened, which can easily lead to exophoria or exotropia. In some paragraphs herein, the concepts represented by the term "myopia" and the term "myopic eye" are indistinguishable and the two can be interchanged. Researchers in this field should correctly understand the meaning of "myopia" or "myopic eye" in this patent through its context.

There are four common classifications of myopia: (1) depending on the refraction, myopia can be divided into mild (300 degrees and below), moderate (300 degrees-600 degrees) and high (600 degrees or above -6D); (2) depending on whether the refractive component is abnormal, myopia can be divided into refractive myopia and axial myopia; (3) depending on whether pathological changes occur, myopia can be divided into pathological myopia and simple myopia; (4) depending on the cause classification, myopia can be divided into primary myopia and complicated/secondary myopia.

For "mild or moderate myopia", the patient has no other symptoms except blurred distant objects, that is, the main problem is that the distance vision gradually decreases, the distant objects are blurred, and the near vision is normal. Usually, the eyes have no special changes except the axial length.

For "high myopia" (such as -6D), the patient's anterior chamber is deeper, the pupil is larger, and the eyeball appears slightly protruding due to the long front-to-back axis. White or gray-white crescent-shaped spots (called myopic semilunar spots) can be seen on the temporal side of the optic disc. This is because the sclera stretches backward and the retinal pigment epithelium and choroid are separated from the temporal edge of the optic disc, exposing the sclera or part of the choroid and sclera. The sclera at the posterior pole continues to expand backward, and geniculate stripes and subretinal new blood vessels may appear in the macula. Patchy atrophic degeneration may occur in the nearby retina and choroid, leading to posterior scleral staphyloma. The macula often has pigment hyperplasia and even bleeding, forming atrophic spots (Forster-Fuchs spots). Such patients are often accompanied by vitreous liquefaction and turbidity, and a few may also develop retinal detachment and complicated cataracts. In addition, high myopia has poor far and near vision, sometimes accompanied by floating black shadows in front of eyes, often due to refractive stromal opacity and retinal and choroidal degeneration. If such lesions occur, this type of myopia is no longer simple myopia.

"Refractive myopia" is mainly due to the excessive curvature of the cornea or lens, the refractive power exceeding the normal range, while the axial length is within the normal range.

"Axial myopia" means that the axial length exceeds the normal range, while the curvature of the cornea and lens is basically within the normal range. Axial myopia is the main type of myopia in children and adolescents.

"Pathological myopia", also known as degenerative myopia, is a degenerative disease of the fundus. These patients with myopia usually have high refraction (for example, more than 600 degrees), and their visual function is obviously damaged, and their distance vision is even worse. In addition, they also often have abnormalities in their visual field, light perception, and contrast perception, often accompanied by poor night vision (night blindness), floaters, floating objects, flashing sensation, *etc.* Thinning of the retinal pigment epithelium and choroid can be seen in the patient's fundus, often accompanied by symptoms such as retinal pigment epithelial atrophy, choroidal neovascularization, retinal detachment, and macular degeneration. In addition, pathological changes in the fundus are still developing after the development has stopped. This type of myopia can cause blindness when it is serious.

"Simple myopia" refers to the myopia that develops during the development period of the eyeball. The development stops and the myopia also tends to be stable. For example, simple myopia usually occurs during school age, the degree of myopia is below 600 degrees, and the fundus generally has no obvious pathological changes. Simple myopia is also called acquired myopia (myopic eye). This type of myopia develops progressively, and the axial length also gradually increases. Appropriate lenses can be used to correct vision to normal, and other visual function indicators are mostly normal. If not, the myopia is non-simple myopia. Examples of non-simple myopia include pathological myopia, high myopia, myopia in the elderly, *etc.*

"Primary myopia" is the main type of myopia. "Primary" refers to a type of myopia with unknown causes such as the cause and mechanism that cannot be determined using existing diagnostic techniques. During its occurrence and development, there are pathological or physiological functional-structural changes specific to myopia, including congenital myopia and acquired simple myopia.

"Complicated/secondary myopia" refers to eye accommodation dysfunction caused by internal and external factors or temporary myopia that occurs due to abnormal refractive index (such as toxic myopia, drug-induced myopia, traumatic myopia, diabetic myopia, incipient cataract myopia, *etc*.)*.* This type of myopia is mostly characterized by clear predisposing factors and frequent fluctuations in vision. This type of myopia is more common in the elderly.

"Axial simple myopia", sometimes also called simple axial myopia, is a type of simple myopia characterized by an imaging focus in front of the retina due to axial length elongation and/or vitreous chamber depth increase. This type of myopia, in which the refractive tissue (such as the lens) is basically normal, is the most common type of myopia in children and adolescents and mostly occurs in people aged 3 to 26 years old, especially those aged 6 to 18 years old (Paul N Baird, Nat Rev Dis Primers. 2020 Dec 17;6(1):99. And A J Adams, Am J Optom Physiol Opt. 1987 Feb;64(2): 150-2 and Seang-Mei Saw, Ophthalmic Physiol Opt. 2005 Sep;25(5):381-91.).

"Curvature myopia" is myopia caused simply by an increase in the curvature of the cornea or lens.

"Index myopia" is myopia mainly caused by the increase in refractive power due to the increase in the refractive index of aqueous humor and lens and belongs to refractive myopia.

"Progressive myopia" refers to a type of myopia in which the refraction continues to decrease over time or as the individual ages. This type of myopia, if not intervened, would generally eventually develop into high myopia.

"Myopia in children and adolescents" is a kind of simple myopia caused by long-term use of eyes at close range. The vast majority of patients with this type of myopia do not develop pathological myopia. The eyeballs of children or adolescents are in the growth and development stage, have strong accommodation ability, and the stretchability of the sclera is relatively large. When reading, writing and other close work, the eyeball is in a defocused state. Over time, the front and rear axis of the eyeball would become longer, which can easily lead to axial myopia in children and adolescents. If eye habits do not change or medical intervention is not taken for myopia, the degree of myopia in children and adolescents would gradually increase, and the corresponding axial length would further increase. Therefore, as children or adolescents grow, develop and learn, the axial length is always dynamic and the refraction continuously changes towards negative, which is a progressive myopia, rather than a static state like the myopia in adults or the elderly. Therefore, the clinical efficacy evaluation of drugs for treating this type of myopia is based on controlling myopia progression and inhibiting axial length elongation as the main indicators.

"Myopia in the elderly" is usually complicated and easily affected by the aging of body organs and underlying diseases. Myopia in the elderly is often accompanied by symptoms such as changes in blood sugar, hardening of blood vessels, insufficient blood supply, and eye microcirculation disorders, and most of the myopia in the elderly are high myopia. A distinctive feature of adults or elderly patients with myopia is that the degree of myopia generally does not increase with age. The main reason is that the eye growth and development of such individuals has basically stopped, and the axial length no longer changes due to long-term close viewing. Therefore, myopia in the elderly (including adults) and myopia in children and adolescents (juveniles) are two completely different types of diseases, and the two indications need to be clearly distinguished in myopia treatment methods. Although the drugs or methods of the present application are suitable for treating various types of myopia, simple myopia is preferred, and non-simple myopia such as myopia in the elderly, high myopia, pathological myopia, *etc.* are excluded, because the clinical intervention strategy for these myopia patients is no longer to suppress axial length elongation, but more likely to prevent blindness caused by serious eye lesions, such as blindness caused by retinal detachment.

"Related symptoms of myopia" include complications caused by myopia, such as complications of high myopia, floaters, glaucoma, posterior scleral staphyloma, retinal detachment, retinal tear, amblyopia, macular hemorrhage, choroidal neovascularization, choroidal atrophy, macular degeneration or maculopathy, visual field defect, progressive or sudden decrease in vision (especially near vision), eye soreness and/or pain, poor night vision (such as night blindness), astigmatism, anisometropia, blindness, vitreous liquefaction, vitreous opacity, squint, frequent blinking, flashes of light, frequent eye rubbing, anisometropia, blurred vision when looking at distant objects, need to squint or partially close eyelids to see distant objects clearly, headache caused by eye strain, difficulty seeing while driving, especially at night (nocturnal myopia), retinal atrophy and degeneration (hemorrhages and holes), subretinal neovascularization, and eyeball atrophy; fundus changes of varying degrees may also occur, such as myopia arc plate, macular hemorrhage or formation of subretinal neovascularization, irregular white atrophic spots, or round black spots with pigmentation (Fuchs spots), and lattice-like degeneration in the periphery of the retina., cystic degeneration, vitreous liquefaction occurring at a younger age, opacity and posterior vitreous detachment, *etc.,* a higher than normal risk of developing retinal holes and detachments, scleral staphyloma often due to long front and back diameter of the eyeball, protruding eyeball, and extreme dilatation of the posterior part of the eyeball.

"Accommodative spastic myopia" is myopia caused by accommodative tension or accommodative spasm due to overload of the eyeball's near vision and excessive accommodation of the ciliary muscles.

"Astigmatic myopia" is a type of myopia in which the cornea is damaged or has varying degrees of curvature, so that external light is diffused in all directions and cannot form a focus on the retina.

In some embodiments, the myopia of the present invention may or may not include pathological myopia, high myopia, myopia in the elderly, astigmatic myopia. These myopias can be corrected by vinpocetine's function of improving blood flow, and the progression of these myopia can also be prevented, treated or slowed down (controlled) by vinpocetine via unknown mechanism.

Myopia intervention means can be divided into myopia correction and myopia treatment. Among them, myopia treatment has the main feature and purpose of inhibiting (controlling) the progression of myopia.

"Myopia correction" is to correct myopia or reduce the degree of myopia through optical methods. Commonly used correction means include wearing framed glasses, transparent lens phacoemulsification, intraocular lens implantation, corneal laser surgery, *etc.* However, this means only corrects the individual refraction and cannot delay the progression of myopia or reverse the excessively long eye axis. In other words, this type of myopia intervention method would not stop or slow down the process of refraction change towards negative continuously, and cannot inhibit the progression of myopia. Therefore, myopia correction methods such as corneal laser surgery can compensate for the patient's refractive error and restore their distance vision. The myopia correction methods are suitable for improving vision in people with pathological myopia, high myopia, or myopia in the elderly, but are not suitable for treating axial myopia or simple myopia, such as myopia in children or adolescents, primary myopia, or mild or moderate myopia. In fact, although the distance vision of individuals with axial myopia temporarily recovers after wearing framed glasses, this correction method would not inhibit the further elongation of the axial length or control the progression rate of myopia. In the absence of other drug treatment, the result is that the degree of myopia would further increase, and patients would need to change to glasses with a larger degree from time to time to continue correcting their vision.

"Inhibit (control) the progression of myopia" refers to using various methods or drugs to delay (slow down) the increase of myopia degree, slow down the rate at which the refraction changes towards negative, control the progression of myopia, and inhibit (control) the continuous elongation of the axial length and belongs to etiological treatment, and most of the patients are juveniles who are still in the stage of eye development. For the treatment of myopia in children or adolescents, slowing down, controlling or even terminating the continuous change towards negative of refraction and inhibiting the corresponding increase in axial length are the primary goals of developing drugs for myopia treatment. That is, the treatment of myopia in children or adolescents with myopia, axial myopia, simple myopia and other types of myopia should achieve the effect of controlling or inhibiting the development of myopia, rather than simply reversing or correcting this refractive error.

Unless otherwise specified, "treat (myopia)" in this application refers to inhibiting (controlling) the progression of myopia, rather than correcting myopia. As used herein, the terms "treat," "slow down", "delay", "inhibit", "control" or "prevent" refer to therapeutic measures and preventive or prophylactic measures aimed at preventing or slowing down (alleviating) or terminating the target conditions or disorders. For example, after receiving a therapeutic dose of vinpocetine compound or a pharmaceutical composition containing the same according to the method described herein, the subject shows observable and/or measured inhibition, delay, reduction and disappearance of one or more symptoms of an ophthalmic condition, or slow down and delay of the progress of the disease, then the subject's ophthalmic condition is successfully treated. It should also be understood that the various modes of treatment or prevention of medical conditions described herein are intended to mean "significant," which includes complete treatment or prevention as well as less than complete treatment or prevention, in which some biologically relevant or medically relevant result is achieved. In some embodiments, "Treatment" does not require 100% elimination of myopia or symptoms of myopia. In some embodiments, "treating" myopia or myopia-related symptoms according to the method of the present application achieves prevention, alleviation, inhibition and arrest of myopia progression, for example, by at least about 5%, at least about 10% or at least about 20%, compared with the level observed in the absence of the composition or method of the present application (for example, in a biologically matched control subject or specimen that has not been exposed to the composition or compound of the present application). In some embodiments, myopia or myopia-related symptoms are treated by at least about 30%, at least about 40%, at least about 50% or at least about 60%, at least about 70%, at least about 80%, at least about 90% or more (about 100%) compared with myopia or myopia-related symptoms in the absence of the compounds of the method of the present application. Therefore, those skilled in the art can understand that "treating", "slowing down", "delaying", "inhibiting", "controlling" or "preventing" do not refer to means beneficial to myopia treatment through an indirect way, such as eye care effects or auxiliary therapeutic effects, for example, enhancing eye blood circulation and improving the comfort of eye application.

The evaluation of the effect of myopia treatment involves many factors, such as whether the sample population is selected scientifically, whether objective controls are set up, and whether an effective dose is administered. Specifically, if the sample population is mostly elderly (eye development has stopped and there may be underlying diseases such as arteriosclerosis), it is difficult to prove that vinpocetine has a therapeutic effect on myopia; if the efficacy of the drug is only evaluated through visual acuity testing without any parallel control (such as a placebo group), it cannot be proven that vinpocetine has a therapeutic effect on myopia. This application uses two classic myopia disease models, guinea pig form deprivation and negative lens induction, which are commonly used in the preclinical development stage of actual myopia drugs, and scientific test standards in this field to evaluate the efficacy of the compound in preventing and controlling myopia, especially preventing and controlling myopia and/or simple myopia in children and adolescents (D A Goss, Am J Optom Physiol Opt. 1981 Oct;58(10):859-69. and Hao Wu, Proc Natl Acad Sci USA. 2018 Jul 24;115(30):E7091-E7100. and Sen Zhang, Invest Ophthalmol Vis Sci. 2019 Jul 1;60(8):3074-3083.).

"Distance vision" is also called naked-eye distance vision. Medically speaking, it refers to visual acuity measured when facing the vision chart at a horizontal distance of 5 meters, with eyes open normally and looking straight ahead, without wearing glasses or any auxiliary equipment that can increase vision (such as framed glasses, contact lenses, color contact lenses, and stenopaic spectacles).

"Local administration" refers to the mode of administration that is directly administrated to the body part to be affected or can achieve the local effect, and includes but not limited to: epidermal administration, inhalation administration, enema administration, ocular administration, and nasal administration.

"Systemic administration" is also called systemic drug delivery, including intravenous administration, oral administration, intramuscular injection, subcutaneous injection and other administration methods. Drugs can be distributed throughout the body through blood transport.

"Improved systemic administration" refers to a safe, efficient and convenient systemic administration method, which can effectively reduce the toxicity of systemic administration and enrich vinpocetine in the eyes at the same time using specific drug targeting technology and the like.

"Direct active ingredient" is also called "active pharmaceutical ingredient", and refers to a substance that has a direct therapeutic effect on a disease (such as myopia). Direct active ingredient is a sufficient condition for the treatment of the disease or its parameters such as the dosage, administration method directly affect the therapeutic effect on the disease, and there is a causal relationship between the administration of the ingredient and the therapeutic effect on the disease.

"Only active ingredient" means that there is and is only one ingredient in the final product (such as a drug) that exerts a medicinal effect, such as directly inhibiting, preventing and/or treating myopia.

"Main active ingredient" refers to an active ingredient whose added amount, proportion, concentration or efficacy, when compared with other active ingredients (such as therapeutic effects on myopia) in the final product (such as medicine), is no less than that of the other active ingredients.

"These substances" refer to vinpocetine, or an optical isomer or a racemate, or a solvate, or a pharmaceutically acceptable salt, or a prodrug, or a metabolite, or a related compound or extract, or a crystalline compound thereof.

In this specification and the appended claims, unless the context clearly indicates otherwise, singular forms, including singular forms "a", "an" and "the/said", also specifically cover plural referents of the terms they refer to. Additionally, as used herein, the word "or" is used in its "inclusive" sense of "and/or" rather than in its "exclusive" sense of "or" unless specifically stated otherwise.

As used herein, reference to a numerical range of a variable is intended to convey that the application may be practiced with the variable equal to any value within that range. Thus, for an intrinsically discontinuous variable, the variable may be equal to any integer value within the stated numerical range, including the endpoints of the range. Similarly, for an intrinsically continuous variable, the variable may be equal to any real value within the stated numerical range, including the endpoints of the range. For example, a variable described as having a numerical value between 0 and 2 may be 0, 1, or 2 for an intrinsically discontinuous variable, and may be 0.0, 0.1, 0.01, 0.001, or any other real value for an intrinsically continuous variable.

As used herein, "about" would be understood by those of ordinary skill in the art and would vary to some extent depending on the context in which it is used. If the use of this term is unclear to those of ordinary skill in the art, "about" in the context in which this term is used would mean a value within plus or minus 10% of the recited value.

As used herein, "administration" of a compound, preparation, or drug to a subject includes any route by which the compound is introduced into or administered to the subject to perform its intended function. "Administration" may be performed by any suitable route, including oral, intraocular, ocular surface, intranasal, parenteral (by intravenous, intramuscular, intraperitoneal or subcutaneous) or local administration (such as local administration to the skin around the eyes). "Administration" includes self-administration and administration by another person. For ocular or periocular administration, the drug may be administered to the affected eye, the unaffected eye, or both of the subject.

As used herein, the term "effective amount" refers to an amount sufficient to obtain the desired therapeutic and/or prophylactic effect, such as to lead to prevention or alleviation of a condition associated with an ophthalmic condition. The amount of the composition administered to the subject would depend on the type and severity of the disease and the properties of the individual, such as general health, age, sex, weight and tolerance to the drug. The amount also depends on the extent, severity and type of the disease. A professional technician would be able to determine the appropriate dosage based on these and other factors. The compositions may also be administered in combination with one or more other therapeutic compounds. In the methods described herein, a vinpocetine compound or a pharmaceutical composition containing the same may be administered to a subject having one or more symptoms or signs of an ophthalmic condition. For example, a "therapeutically effective amount" of vinpocetine is an average level of the pharmacological effect that minimally alleviates the ophthalmic condition.

As used herein, the terms "preparation" and "composition" are used interchangeably and refer to a mixture of two or more compounds, elements, or molecules. In some aspects, the terms "preparation" and "composition" may be used to refer to a mixture of one or more active agents with a carrier or other excipients. The composition can take virtually any physical form, including solid, liquid (for example, solution), or gas.

Furthermore, the term "dosage form" may include one or more preparations or compositions provided in a form for administration to a subject. For example, an injectable dosage form may be a preparation or composition prepared in a manner suitable for administration by injection.

As used herein, the term "pharmaceutically acceptable" means a carrier, concentration, or existing form of a drug for animals, preferably for humans, which is approved by regulatory authorities such as CFDA (China), EMEA (Europe) and/or FDA(US) and/or any other national regulatory authorities.

As used herein, the term "simultaneous" therapeutic application refers to the administration of at least two active ingredients (compounds) by the same route and at the same time or substantially at the same time.

As used herein, the term "separate" therapeutic application refers to the administration of at least two active ingredients (compounds) by different routes at the same time or substantially at the same time.

As used herein, the term "sequential" therapeutic application refers to the administration of at least two active ingredients at different times, by the same or different administration routes. More specifically, sequential application refers to the complete administration of one of the active ingredients before the administration of the other active ingredients begins. Thus, one active ingredient may be administered minutes, hours or days before the other active ingredients are administered. No concurrent treatment is performed in this case.

As used herein, the term "preventing" a disorder or condition refers to a compound that, in a statistical sample, reduces the onset of a disorder or condition in a treated sample relative to an untreated control sample, or delays the onset of one or more symptoms of a disorder or condition in a treated sample relative to an untreated control sample or alleviates the severity of one or more symptoms of a disorder or condition.

As used herein, the term "related compounds" includes, but is not limited to, derivatives or structural analogs of vinpocetine.

As used herein, the term "derivative" or "analog" of a compound includes any molecule functionally and/or structurally related to the compound, such as acids, amides, esters, ethers, acetylated variants, hydroxylated variants or alkylated variants of the compound. The term derivative also includes structurally related compounds that have lost one or more of the substituents listed above. Preferred derivatives of a compound are molecules that have a significant degree of similarity to the compound, as determined by known methods. Similar compounds together with their similarity index to the parent molecule can be found in a large number of databases, such as PubChem (http://pubchem.ncbi.nlm.nih.gov/search/) or DrugBank (http://www.drugbank.ca/). In a more preferred embodiment, the derivative should have a Tanimoto similarity index to the parent drug of greater than 0.4, preferably greater than 0.5, more preferably greater than 0.6, even more preferably greater than 0.7. The Tanimoto similarity index is widely used to measure the degree of structural similarity between two molecules. The Tanimoto similarity index can be calculated by software available online such as Small Molecule Subgraph Detector (http://www.ebi.ac.uk/thomton-srv/software/SMSD/). Preferred derivatives should be structurally and functionally related to the parent compound, *i.e*., they should also retain at least part of the activity of the parent drug. Furthermore, the term "derivative" also includes a metabolite of a drug, for example, the molecule is usually produced by (biochemical) modification or processing of the drug through a special catalytic system after being administered to an organism, and shows or retains the biological activity of the drug. Furthermore, the term "derivative" also includes halogenated (for example, fluorinated), protonated, deuterated, tritiated compounds or a combination thereof of vinpocetine or its salt form.

In specific embodiments, as used herein, "metabolite" refers to a modified or processed drug that retains at least part of the activity of the parent drug, preferably has an inhibitory effect on phosphodiesterase activity or has a therapeutic, preventive or slowing effect on myopia and related symptoms.

"Extracts" or "plant extracts" refer to substances extracted or processed from a plant (all or a part of the plant) as a raw material using appropriate chemical or physical methods, such as solvent extraction or pressing. The substances can be pure, or contain small amounts of impurities, or is a mixture.

"Ophthalmic composition" or "ocular preparation" or "ophthalmic preparation" refers to an ophthalmic composition, or an ophthalmic pharmaceutical composition, or an ophthalmic pharmaceutical product; or drugs, preparations, cosmetics, health products, drug-device combinations or devices used to prevent and/or treat eye diseases, protect, maintain and improve vision, and avoid, slow down or reverse vision damage; or eye drops (eyewash), eye ointments, eye sprays, implant tablets, eye gels, eye patches, eye microspheres, eye sustained-release preparations, periorbital injections, intraocular injections, *etc.*

"Fish oil" refers to lipids derived from higher animals, especially fish (such as cod, salmon), squid, and seals, especially polyunsaturated fatty acids, including but not limited to Omega-3 unsaturated fatty acids, DHA, EPA, DPA, ALA, nisinic acid, stearidonic acid, eicosatetraenoic acid or a combination thereof.

The present application discloses the identification of a compound, namely vinpocetine, for use in the treatment, prevention or slowing down of myopia and its associated symptoms, to enhance myopia reduction or myopia slowing down effects while avoiding or minimizing adverse side effects, such as adverse side effects observed by atropine therapy.

The present application relates to a pharmaceutical composition or a method for treating, preventing, or slowing down myopia and its associated symptoms in an individual such as a toddler, school-age child, adolescent, or younger adult. In some examples, treating, preventing, or slowing down myopia and related symptoms may include administering a therapeutically effective amount of a pharmaceutical composition or dosage form to a subject in need thereof.

The pharmaceutical composition contains a therapeutically effective amount of a vinpocetine compound or a salt thereof or a related compound thereof.

In another aspect, the present application provides an ophthalmic device comprising a pharmaceutical composition comprising vinpocetine or a therapeutically acceptable salt thereof or a derivative thereof, preferably, wherein the ophthalmic device delivers the pharmaceutical composition in a sustained release manner; preferably, wherein said ophthalmic device delivers said pharmaceutical composition in a pulsed manner.

Through experiments, we unexpectedly found that the use of vinpocetine alone can significantly slow down the process of refraction change towards negative in the form deprivation or lens-induced guinea pig myopia model. In some individuals, the process of refraction change towards negative is even completely inhibited (terminated), and axial length elongation can be significantly inhibited. Based on this, it can be confirmed that vinpocetine compound has the effects of preventing or treating, preventing or controlling the progression of myopia in animals, especially humans, such as toddler, school-age children, children and adolescents, juveniles, people aged 3 to 26 or young adults.

In some embodiments, the treatment subjects of the technical solution of the present application are children or adolescents, with an age range of 3-26 years old, preferably 6-18 years old, or 12-18 years old. In some embodiments, the treatment subjects of the technical solution of the present application are adults, for example, aged 16-65 years old, preferably 16-26 years old.

In local administration experiments, no discomfort or eye abnormalities are observed in any administered animals, nor were there any toxic manifestations such as weight loss or decreased food intake. Based on the existing clinical application basis of vinpocetine, those skilled in the art can expect that: vinpocetine would have good drug safety during the clinical treatment of treating, preventing or improving myopia and related symptoms.

The pharmaceutical composition of the present application has a significantly better technical effect than atropine in treating, preventing or slowing down myopia and its related symptoms, and no adverse reactions such as eye irritation, pupil dilation, inflammation or allergy are observed in the experiment.

In certain embodiments of the pharmaceutical composition, ophthalmic device, or treatment method disclosed herein, the patient is treated for a period of time between about 1 month and 20 years, such as a period of time of at least 6 months, at least 1 year, at least 2 years, at least 3 years, at least 5 years, at least 7 years, or at least 9 years.

In one embodiment, in the pharmaceutical composition, ophthalmic device or treatment method of the present application, the pharmaceutical composition comprises vinpocetine and a therapeutically acceptable salt thereof or a derivative thereof, preferably, the pharmaceutical composition comprises a pharmaceutically acceptable carrier.

In other embodiments, the pharmaceutical composition, ophthalmic device or treatment method according to any one of the above embodiments and any one or more of the other embodiments herein, wherein the pharmaceutical composition is an aqueous composition.

In other embodiments, the pharmaceutical composition, ophthalmic device or treatment method according to any one of the above embodiments and any one or more of the other embodiments herein, wherein the pharmaceutical composition is an ophthalmic preparation.

In other embodiments, the pharmaceutical composition, ophthalmic device or treatment method according to any one of the above embodiments and any one or more of the other embodiments herein, wherein the pharmaceutical composition is an ophthalmic aqueous preparation.

In other embodiments, the pharmaceutical composition, ophthalmic device or treatment method according to any one of the above embodiments and any one or more of the other embodiments herein, wherein the pharmaceutical composition is an eye drop preparation.

In other embodiments, the pharmaceutical composition, ophthalmic device or treatment method according to any one of the above embodiments and any one or more of the other embodiments herein, wherein the pharmaceutical composition is an eye spray preparation. In other embodiments, the pharmaceutical composition, ophthalmic device or treatment method according to any one of the above embodiments and any one or more of the other embodiments herein, wherein the pharmaceutical composition is an ocular pharmaceutical composition contained in a contact lens blister package.

In other embodiments, the pharmaceutical composition, ophthalmic device or treatment method according to any one of the above embodiments and any one or more of the other embodiments herein, wherein the pharmaceutical composition is a local preparation.

In other embodiments, the pharmaceutical composition, ophthalmic device or treatment method according to any one of the above embodiments and any one or more of the other embodiments herein, wherein the pharmaceutical composition is an ophthalmic composition.

In other embodiments, the pharmaceutical composition, ophthalmic device or treatment method according to any one of the above embodiments and any one or more of the other embodiments herein, wherein the pharmaceutical composition is a local ophthalmic composition.

In other embodiments, the pharmaceutical composition, ophthalmic device or treatment method according to any one of the above embodiments and any one or more of the other embodiments herein, wherein the pharmaceutical composition is an ophthalmic gel preparation.

In other embodiments, the pharmaceutical composition, ophthalmic device or treatment method according to any one of the above embodiments and any one or more of the other embodiments herein, wherein the pharmaceutical composition is an ophthalmic emulsion.

In other embodiments, the pharmaceutical composition, ophthalmic device or treatment method according to any one of the above embodiments and any one or more of the other embodiments herein, wherein the pharmaceutical composition is an ophthalmic lipidosome.

In other embodiments, the pharmaceutical composition, ophthalmic device or treatment method according to any one of the above embodiments and any one or more of the other embodiments herein, wherein the pharmaceutical composition is a nanodisc.

In other embodiments, the pharmaceutical composition, ophthalmic device or treatment method according to any one of the above embodiments and any one or more of the other embodiments herein, wherein the pharmaceutical composition is a nanoparticle suspension.

In other embodiments, the pharmaceutical composition, ophthalmic device or method of treatment according to any one or more of the above embodiments and any one or more of the other embodiments herein, wherein the pharmaceutical composition is a salve or ointment or unguent.

In other embodiments, the pharmaceutical composition, ophthalmic device or treatment method according to any one of the above embodiments and any one or more of the other embodiments herein, wherein the pharmaceutical composition is an ophthalmic ointment (such as an eye ointment).

In other embodiments, the drug, preparation, composition, device or treatment method according to any one of the above embodiments and any one or more of the other embodiments herein, wherein the drug, preparation and composition is an oral preparation, such as a tablet or capsule or a powder or syrup.

In other embodiments, the drug, preparation, composition, device or treatment method according to any one of the above embodiments and any one or more of the other embodiments herein, wherein the drug, preparation and composition further comprise one or more other ophthalmologically acceptable excipients and additives, including carriers, stabilizers, osmotic pressure regulators, preservatives, antioxidants, buffers, tonicity regulators, thickeners or other excipients.

In other embodiments, the drug, preparation, composition, device or treatment method according to any one of the above embodiments and any one or more of the other embodiments herein, wherein the drug, preparation, composition, device or treatment method comprises a polyhydric alcohol and/or an acidic co-solvent, optionally hydroxypropyl-beta-cyclodextrin and citric acid or tartaric acid.

In other embodiments, the drug, preparation, composition, device or treatment method according to any one of the above embodiments and any one or more of the other embodiments herein, wherein the carriers are selected from but not limited to: water, mixtures of water and water-miscible solvents, vegetable or mineral oil containing 0.01% to 5% by weight of hydroxyethyl cellulose, ethyl oleate, carboxymethocel, carboxymethyl cellulose, hydroxymethylcellulose, hydroxyethyl cellulose, ethyl acrylate, polyacrylamide, pectin, alginate, starch derivatives, Polyvinyl alcohol, polyvinylpyrrolidone, polyvinyl methyl ether, polyethylene oxide, cross-linked polyacrylic acid, carbopol, lecithin, polyethylene glycol stearate, polyhydric alcohol, acidic co-solvent, heptadecanethyleneoxyoxohexadecanol or polyoxyethylene sorbitol monooleate.

In other embodiments, the drug, preparation, composition, device or treatment method according to any one of the above embodiments and any one or more of the other embodiments herein, wherein the osmotic pressure regulator is sodium chloride.

In other embodiments, the drug, preparation, composition, device or treatment method according to any one of the above embodiments and any one or more of the other embodiments herein, wherein the preservatives are selected from benzalkonium chloride, cetrimonium, sodium perborate, stable oxychloride complex, Sofia, polyquatemium-1, chlorobutanol, disodium ethylenediamine tetraacetate, polyhexamethylene biguanide or a combination thereof.

In other embodiments, the drug, preparation, composition, device or treatment method according to any one of the above embodiments and any one or more of the other embodiments herein, wherein the buffers are selected from borates, borate-polyol complexes, phosphate buffers, citrate buffers, acetate buffers, carbonate buffers, organic buffers, amino acid buffers, or combinations thereof.

In other embodiments, the drug, preparation, composition, device or treatment method according to any one of the above embodiments and any one or more of the other embodiments herein, wherein the tonicity regulators are selected from sodium chloride, sodium nitrate, sodium sulfate, sodium bisulfate, potassium chloride, calcium chloride, magnesium chloride, zinc chloride, potassium acetate, sodium acetate, sodium bicarbonate, sodium carbonate, sodium thiosulfate, magnesium sulfate, disodium hydrogen phosphate, sodium dihydrogen phosphate, potassium dihydrogen phosphate, glucose, mannitol, sorbitol, glucose, sucrose, urea, propylene glycol, glycerol or a combination thereof.

In other embodiments, when related to the use of the present application, the prevention and control (treatment) effect on myopia or the inhibition of axial length by vinpocetine, or an optical isomer or a racemate, or a solvate, or a pharmaceutically acceptable salt, or a prodrug, or a metabolite, or a related compound or extract, or a crystalline compound thereof, or a combination of these substances have nothing to do with the sex, age, type of myopia, progression speed of myopia and severity of myopia.

In other embodiments, the pharmaceutical composition, ophthalmic device or treatment method according to any one of the above embodiments and any one or more of the other embodiments herein, wherein the pharmaceutical composition is a sustained release preparation or subconjunctival depot.

In other embodiments, the pharmaceutical composition, ophthalmic device or treatment method according to any one of the above embodiments and any one or more of the other embodiments herein, wherein the pharmaceutical composition is a sustained release preparation.

In other embodiments, the pharmaceutical composition, ophthalmic device or treatment method according to any one of the above embodiments and any one or more of the other embodiments herein, wherein the pharmaceutical composition is a sustained release preparation comprised in an ophthalmic device.

In other embodiments, the pharmaceutical composition, ophthalmic device or treatment method according to any one of the above embodiments and any one or more of the other embodiments herein, wherein the pharmaceutical composition is comprised in an ophthalmic device, such as an eye patch, a suture line, and a corneal contact lens.

In other embodiments, the pharmaceutical composition, ophthalmic device or treatment method according to any one of the above embodiments and any one or more of the other embodiments herein, wherein the pharmaceutical composition is an ophthalmic composition and the ophthalmic composition is comprised in an ophthalmic device.

In other embodiments, the pharmaceutical composition, ophthalmic device or treatment method according to any one of the above embodiments and any one or more of the other embodiments herein, wherein the ophthalmic device is a contact lens, an ocular insert, a corneal covering, a corneal inlay, a nanodisc, a liposome, a nanoparticle, a punctal plug, or a hydrogel matrix with a microfluidic depot.

In other embodiments, the pharmaceutical composition, ophthalmic device or treatment method according to any one of the above embodiments and any one or more of the other embodiments herein, wherein the ophthalmic device is a contact lens.

In other embodiments, the pharmaceutical composition, ophthalmic device or treatment method according to any one of the above embodiments and any one or more of the other embodiments herein, wherein the ophthalmic device is an ocular insert.

In other embodiments, the pharmaceutical composition, ophthalmic device or treatment method according to any one of the above embodiments and any one or more of the other embodiments herein, wherein the ophthalmic device is a corneal covering.

In other embodiments, the pharmaceutical composition, ophthalmic device or treatment method according to any one of the above embodiments and any one or more of the other embodiments herein, wherein the ophthalmic device is a corneal inlay.

In other embodiments, the pharmaceutical composition, ophthalmic device or treatment method according to any one of the above embodiments and any one or more of the other embodiments herein, wherein the ophthalmic device is a nanodisc.

In other embodiments, the pharmaceutical composition, ophthalmic device or treatment method according to any one of the above embodiments and any one or more of the other embodiments herein, wherein the ophthalmic device is a liposome.

In other embodiments, the pharmaceutical composition, ophthalmic device or treatment method according to any one of the above embodiments and any one or more of the other embodiments herein, wherein the ophthalmic device is a nanoparticle.

In other embodiments, the pharmaceutical composition, ophthalmic device or treatment method according to any one of the above embodiments and any one or more of the other embodiments herein, wherein the ophthalmic device is a punctal plug.

In other embodiments, the pharmaceutical composition, ophthalmic device or treatment method according to any one of the above embodiments and any one or more of the other embodiments herein, wherein the ophthalmic device is a hydrogel matrix with a microfluidic depot.

In other embodiments, the pharmaceutical composition, ophthalmic device or treatment method according to any one of the above embodiments and any one or more of the other embodiments herein, wherein the ophthalmic device delivers the pharmaceutical composition in a sustained release manner.

In other embodiments, the pharmaceutical composition, ophthalmic device or treatment method according to any one of the above embodiments and any one or more of the other embodiments herein, wherein the pharmaceutical composition is formulated as an ophthalmic composition for the treatment of ophthalmic disorders or conditions.

In other embodiments, the pharmaceutical composition, ophthalmic device or treatment method according to any one of the above embodiments and any one or more of the other embodiments herein, wherein the pharmaceutical composition is formulated as an ophthalmic composition for treating pre-myopia, myopia, true myopia, pseudomyopia or myopia progression.

In other embodiments, the pharmaceutical composition, ophthalmic device or treatment method according to any one of the above embodiments and any one or more of the other embodiments herein, wherein the pharmaceutical composition is formulated as an ophthalmic composition for treating high myopia, moderate myopia or low myopia.

In other embodiments, the pharmaceutical composition, ophthalmic device or treatment method according to any one of the above embodiments and any one or more of the other embodiments herein, Wherein the pharmaceutical composition is formulated as an ophthalmic composition for treating patients diagnosed with pre-myopia (or at risk of developing myopia).

In other embodiments, the pharmaceutical composition, ophthalmic device or treatment method according to any one of the above embodiments and any one or more of the other embodiments herein, wherein the pharmaceutical composition is substantially uniformly distributed throughout the ophthalmic device.

In other embodiments, the pharmaceutical composition, ophthalmic device or treatment method according to any one of the above embodiments and any one or more of the other embodiments herein, wherein the ophthalmic device is comprised in a contact lens blister package.

In other embodiments, the pharmaceutical composition, ophthalmic device or treatment method according to any one of the above embodiments and any one or more of the other embodiments herein, wherein the pharmaceutical composition is immersed in an ophthalmic device comprised in a contact lens blister package.

Pharmaceutical preparation form, administration mode and dosage: Any method known to those skilled in the art may be used to contact cells, organs or tissues with the vinpocetine compound. Suitable methods include *in vitro, ex vivo* or *in vivo* methods. *In vivo* methods generally include administering the vinpocetine compound of the present application or a pharmaceutical composition containing the same to a mammal, preferably to a human. When used *in vivo* for treatment, the vinpocetine compound or a pharmaceutical composition containing the same can be administered to a subject in an effective amount (*i*.*e*., an amount having the desired therapeutic effect). Dosage and dosing regimen would depend on the extent of the ophthalmic condition in a subject, the subject, and the subject's medical history.

The compounds disclosed herein may also exist as a prodrug. The prodrug of the compound described herein is a modified form that are susceptible to chemical changes under physiological conditions to yield vinpocetine or its related compounds. In addition, the prodrug can be converted to the compound in an *ex vivo* environment by chemical or biochemical methods. For example, the prodrug can be slowly converted to the compound when placed in a transdermal patch reservoir with appropriate enzymes or chemical reagents. The prodrug is often useful because, in some cases, it may be easier to administer than the compound or parent drug. For example, the prodrug is bioavailable through oral administration, whereas the parent drug is not. the prodrug can also be more soluble in the pharmaceutical composition than the parent drug. Many prodrug derivatives are known in the art, such as those that rely on hydrolytic cleavage or oxidative activation of the prodrug. One non-limiting example of the prodrug is a compound that is administered as an ester ("prodrug") but is then metabolically hydrolyzed to a carboxylic acid (the active entity).

The compounds disclosed herein may exist as therapeutically acceptable salts. This application includes the compounds listed above in salt form, including acid addition salts. Suitable salts include those formed with organic and inorganic acids. Such acid addition salts are generally pharmaceutically acceptable. However, non-pharmaceutically acceptable salts can be used to prepare and purify problematic compounds. Base addition salts may also be formed and are pharmaceutically acceptable.

As used herein, the term "therapeutically acceptable salt" means a salt or zwitterionic form of the compound disclosed herein that is water- or oil-soluble or dispersible and therapeutically acceptable, as defined herein. Salts can be prepared during the final isolation and purification of the compound, or prepared separately by reacting the appropriate free base form of the compound with a suitable acid. Representative acid addition salts include acetate, adipate, alginate, L-ascorbate, aspartate, benzoate, benzenesulfonate, besylate, bisulfate, butyrate, camphorate, camphorsulfonate, citrate, digluconate, formate, fumarate, gentisate, glutarate, glycerophosphate, glycolate, hemisulfate, heptylate, caproate, hippurate, hydrochloride, hydrobromide, hydroiodide, 2-hydroxyethanesulfonate (Isethionate), Lactate, maleate, malonate, DL-mandelate, mesitylenesulfonate, mesylate, naphthalenesulfonate, nicotinate, 2-naphthalene sulfonate, oxalate, pamoate, pectinate, persulfate, 3-phenylpropionate, phosphonate, citrate, picrate, pivalate, propionate, pyroglutamate, succinate, sulfonate, tartrate, L-tartrate, trichloroacetate, trifluoroacetate, phosphate, glutamate, bicarbonate, para-toluenesulfonate (p-tosylate) and undecanoate. Additionally, basic groups in the compounds disclosed herein may be quaternized with: methyl, ethyl, propyl and butyl chlorides, bromides and iodides; dimethyl, diethyl, dibutyl and dipentyl sulfates; decyl, lauryl, myristyl and steryl chlorides, bromides and iodides; and benzyl and phenethyl bromides. Examples of acids that can be used to form therapeutically acceptable addition salts include inorganic acids (such as hydrochloric acid, hydrobromic acid, sulfuric acid, and phosphoric acid) and organic acids (such as oxalic acid, maleic acid, succinic acid, and citric acid). Salts can also be formed by coordination of the compounds with alkali metal or alkaline earth metal ions. Accordingly, this application includes the sodium, potassium, magnesium and calcium salts, and the like, of the compounds disclosed herein.

The crystalline forms of vinpocetine include all known species, such as the crystalline form described by Samuel Golob in the literature (Samuel Golob, Improving Biopharmaceutical Properties of Vinpocetine Through Cocrystallization, J Pharm Sci. 2016 Dec;105(12):3626-3633.).

Base addition salts can be prepared by reacting carboxyl groups with suitable bases (such as hydroxides, carbonates or bicarbonates of metal cations) or with ammonia or organic primary, secondary or tertiary amines during the final separation and purification of the compounds. Cations of therapeutically acceptable salts include lithium, sodium, potassium, calcium, magnesium, and aluminum and nontoxic quaternary ammonium cations such as ammonium, tetramethylammonium, tetraethylammonium, methylamine, dimethylamine, trimethylamine, triethylamine, diethylamine, ethylamine, tributylamine, pyridine, N,N-dimethylaniline, N-methylpiperidine, N-methylmorpholine, dicyclohexylamine, procaine, dibenzylamine, N,N-dibenzylphenethylamine, 1-diphenyldroxymethylamine and N,N'- dibenzylethylenediamine. Other representative organic amines suitable for forming base addition salts include ethylenediamine, ethanolamine, diethanolamine, piperidine, and piperazine.

Although it is possible to administer the compounds of the present application in the form of crude chemicals, it is also possible to provide them as pharmaceutical preparations. Accordingly, provided herein are a pharmaceutical preparation comprising one or more certain compounds disclosed herein, or one or more pharmaceutically acceptable salts, esters, prodrugs, amides or solvates thereof, together with one or more pharmaceutically acceptable carrier and optionally one or more other therapeutic ingredients. A carrier is "acceptable" in the sense of being compatible with the other ingredients of the preparation and not deleterious to the recipient thereof. Proper preparation depends on the chosen route of administration. Any known technique, carrier and excipient that is suitable and understood in the art may be used; See for example Remington's Pharmaceutical Sciences. The pharmaceutical composition disclosed herein may be produced in any manner known in the art, for example by means of conventional mixing, dissolving, granulating, sugar-coating, grinding, emulsifying, encapsulating, embedding or compression processes, and the pharmaceutical composition may also be made as a combination of medicine and device.

The preparation includes a preparation suitable for oral, parenteral (including subcutaneous, intradermal, intramuscular, intravenous, intraarticular and intramedullary), intraperitoneal, transmucosal, transdermal, rectal and local (including dermal, buccal, sublingual, ocular, intranasal and intraocular) administration, the most appropriate route may depend, for example, on the disorder and condition of the recipient. The preparation may be conveniently presented in unit dosage form and may be prepared by any method known in the art of pharmacy. Typically, these methods involve a step of combining the compound of the present application, or a pharmaceutically acceptable salt, ester, amide, prodrug or solvate thereof (the "active ingredient") with a carrier that constitutes one or more accessory ingredients. In general, the active ingredient is uniformly and closely combined with a liquid carrier or a finely divided solid carrier or both, and then the product is shaped into a desired preparation if necessary, thereby preparing the preparation.

The preparation of the compound disclosed herein suitable for oral administration may be provided in discrete units such as capsules, cachets, or tablets, each containing a predetermined amount of the active ingredient; provided as powder or granules; provided as a solution or suspension in an aqueous or non-aqueous liquid; or provide as an oil-in-water emulsion or a water-in-oil emulsion. The active ingredient may also be presented as a bolus, electuary or paste.

The pharmaceutical preparations for oral use include tablets, push-fit capsules made of gelatin, and sealed soft capsules made of gelatin and a plasticizer such as glycerin or sorbitol. Tablets may be prepared by compression or molding, optionally with one or more accessory ingredients. Compressed tablets may be prepared by compressing in a suitable machine the active ingredient in a free-flowing form such as powders or granules, optionally mixed with a binder, inert diluent or lubricant, surfactant or dispersing agent. Molded tablets may be prepared by molding in a suitable machine a mixture of the powdered compound moistened with an inert liquid diluent. The tablets may optionally be coated or scored and may be formulated so as to provide sustained or controlled release of the active ingredient therein. The dosage of all preparations for oral administration should be suitable for the purpose of myopia prevention or treatment for which such administration is intended. Push-fit capsules may contain the active ingredients mixed with fillers (such as lactose), binders (such as starch) and/or lubricants (such as talc or magnesium stearate), and optionally stabilizers. In soft capsules, the active compounds can be dissolved or suspended in suitable liquids, such as fatty oils, liquid paraffin, or liquid polyethylene glycols. In addition, stabilizers can be added. Dragee cores with a suitable coating are provided. For this purpose, concentrated sugar solutions can be used, which may optionally contain gum arabic, talc, polyvinylpyrrolidone, carbopol gel, polyethylene glycol and/or titanium dioxide, lacquer solutions and suitable organic solvents or solvent mixtures. Dyestuffs or pigments may be added to the tablets or dragee coatings to identify or characterize different combinations of active compound doses.

Examples of fillers or diluents used in oral pharmaceutical preparations such as capsules and tablets include, but are not limited to, lactose, mannitol, xylitol, dextrose, sucrose, sorbitol, compressible sugar, microcrystalline cellulose (MCC), powdered cellulose, corn starch, pregelatinized starch, glucose binder (dextrate), dextran, dextrin, dextrose, maltodextrin, calcium carbonate, calcium hydrogen phosphate, tricalcium phosphate, calcium sulfate, magnesium carbonate, magnesium oxide, poloxamer (e.g., polyethylene oxide), methyl cellulose and hydroxypropyl methyl cellulose. The filler may have complexed solvent molecules, as in the case where the lactose used is lactose monohydrate.

Examples of disintegrants used in oral pharmaceutical preparations such as capsules and tablets include, but are not limited to, sodium starch glycolate, sodium carboxymethyl cellulose, calcium carboxymethyl cellulose , croscarmellose sodium, povidone, Crospovidone (polyvinylpolypyrrolidone), methylcellulose, microcrystalline cellulose, powdered cellulose, low substituted hydroxypropyl cellulose, starch, pregelatinized starch and sodium alginate.

In addition, glidants and lubricants may be used in oral pharmaceutical preparations to ensure uniform blending of the excipients during mixing. Examples of lubricants include, but are not limited to, calcium stearate, glycerin monostearate, glyceryl palmitate stearate, hydrogenated vegetable oil, light mineral oil, magnesium stearate, mineral oil, polyethylene glycol, sodium benzoate, sodium lauryl sulfate, sodium stearyl fumarate, stearic acid, talc and zinc stearate. Examples of glidants include, but are not limited to, silica (SiO₂), talc cornstarch, and poloxamer. Poloxamer (or available from BASF Corporation) is a A-B-A block copolymer where the A segment is a hydrophilic polyethylene glycol homopolymer and the B segment is a hydrophobic polypropylene glycol homopolymer.

Examples of tablet binders include, but are not limited to, gum arabic, alginic acid, carbomer, sodium carboxymethyl cellulose, dextrin, ethyl cellulose, gelatin, guar gum, hydrogenated vegetable oil, hydroxyethyl cellulose, hydroxypropyl cellulose, hydroxypropyl methylcellulose, copolyvidone, methylcellulose, liquid glucose, maltodextrin, polymethacrylate, povidone, pregelatinized starch, sodium alginate, starch, sucrose, tragacanth and corn protein.

The compounds may be formulated for parenteral administration by injection, for example by bolus injection or continuous infusion. Preparations for injection may be provided in unit dosage form, for example, in ampoules or multi-dose containers with an added preservative. The compositions may take forms such as suspension, solution or emulsion in oily or aqueous vehicles, and may contain formulation agents such as suspending, stabilizing and/or dispersing agents. The preparations may be provided in unit-dose or multi-dose containers, such as sealed ampoules and vials, and may be stored in powder form or in a freeze-dried (lyophilized) state, which only needs to add sterile liquid carriers, such as physiological saline or sterile and nonpyrogenic water, just before use. Ready-to-use injection solutions and suspensions may be prepared from sterile powders, granules, and tablets of the types previously described. In a preferred embodiment, the pharmaceutical composition of the present application is in the form of an injection, especially a syringe. Preferably, the pharmaceutical composition is administered by intraocular injection, more preferably by intravitreal injection into the vitreous body.

The preparations for parenteral administration include, but are not limited to: aqueous and non-aqueous (oily) sterile injectable solutions of the active compounds, which may contain antioxidants, buffers, bacteriostatic agents and solutes rendering the preparations isotonic with the blood of the intended recipient; and aqueous and non-aqueous sterile suspensions, which may contain suspending agents and thickeners. Suitable lipophilic solvents or vehicles include fatty oils (such as sesame oil) or synthetic fatty acid esters (such as ethyl oleate or triglycerides) or liposomes. Aqueous injection suspensions may contain substances that increase the viscosity of the suspension, such as methylcellulose, sodium carboxymethylcellulose, sorbitol, or dextran. Optionally such as ethyl oleate or triglyceride) or liposomes. Aqueous injection suspensions may contain substances that increase the viscosity of the suspension, such as sodium carboxymethylcellulose, sorbitol, or dextran. Optionally, the suspensions may also contain suitable stabilizers or regents that increase the solubility of the compounds to enable the formulation of highly concentrated solutions.

In addition to the aforementioned preparations, the compounds mentioned in this patent such as vinpocetine can also be formulated into storage preparations. Such long-acting preparations may be administered by implantation (for example, subcutaneously or intramuscularly) or by intramuscular injection. Thus, for example, the compounds may be formulated with suitable polymeric or hydrophobic materials (for example, as an emulsion in an acceptable oil) or ion exchange resins, or formulated as sparingly soluble derivatives, for example, as sparingly soluble salts.

For buccal or sublingual administration, the compositions may take the form of tablets, troches, lozenges or gels formulated in conventional manner. Such compositions may contain active ingredients, such as compounds such as vinpocetine mentioned in this patent, in a flavored matrix such as sucrose and gum arabic or tragacanth.

The compounds may also be formulated as rectal compositions such as suppositories or retention enemas, for example, containing conventional suppository matrix such as cocoa butter, polyethylene glycols, or other glycerides.

In addition to the aforementioned preparations, the compounds mentioned in this patent such as vinpocetine can also be formulated into disposable preparations for local administration, devices, eye appearance-changing products or pharmaceutical consumables, such as single-use eye drops (each package contains only one therapeutically effective dose), individually packaged eye patches, coloured contact lenses, and daily disposable contact lenses.

The compounds mentioned, such as vinpocetine disclosed herein, may be administered locally, that is, by non-systemic administration. This includes applying the compounds disclosed herein to the outside of the eye, epidermis, or mouth, and instilling the compounds into the ears, eyes, and nose so that the compound does not significantly enter the bloodstream. In contrast, systemic administration refers to oral, intravenous, intraperitoneal, and intramuscular administration.

Preparations suitable for local administration include liquid or semi-liquid preparations (such as gels, liniments, lotions, creams, ointments or pastes) suitable for penetrating the skin to the site of action and preparations suitable for application to the eyes, ears or nose, such as drops and sprays. Active ingredients for myopia treatment may account for, for example, 0.0000001% to 99% w/w (by weight) of the total content of the preparation. In certain embodiments, the active ingredient may account for up to 50% w/w or more. In other embodiments, the active ingredient may account for less than 1% w/w. In certain embodiments, the active ingredient may account for 0.01% w/w to 1% w/w. In other embodiments, the active ingredient may account for 0.00001% to 0.001% w/w of the preparation; or, the active ingredient for local administration may account for, for example, 0.0000001% to 100% w/v (by weight to volume) of the preparation; or, in certain embodiments, the active ingredient may account for up to 50% w/v or more. In other embodiments, the active ingredient may account for less than 1% w/v. In certain embodiments, the active ingredient may account for 0.01% w/v to 1% w/v. In other embodiments, the active ingredient may account for 0.00001% to 0.001% w/v of the preparation.

The gel preparation form includes a preparation formed by connecting vinpocetine or prodrugs thereof or related compounds thereof to a hydrogel. Preferably, the hydrogel connected to the vinpocetine or prodrugs thereof is a biological degradable hydrogel.

The hydrogel comprises, preferably consists of, at least one polymer, preferably selected from poly(acrylic acid), poly(acrylate), poly(acrylamide), poly(alkoxy)polymer, poly(amide), poly(amidoamine), poly(amino acid), poly(anhydride), poly(asparagine), poly(butyric acid), poly(caprolactone), poly(carbonate), poly(cyanoacrylate), poly(dimethylacrylamide), poly(ester), poly(ethylene), poly(ethylene glycol), poly(ethylene oxide), poly(ethylazoline), poly(glycolic acid), poly(hydroxyethyl acrylate), poly(hydroxyethylazoline), poly(hydroxypropylmethacrylamide), poly(hydroxypropyl methacrylate), poly(hydroxypropylazoline), poly(iminocarbonate), poly(N-isopropylacrylamide), poly(lactic acid), poly(lactic-co-glycolic acid), poly(methacrylamide), poly(methacrylate), poly(methylazoline), polypropylene glycol fumarate), poly(organophosphorus cyanide), poly(orthoester), poly(azoline), polypropylene glycol), poly(siloxane), poly(urethane), poly(vinyl alcohol), poly(vinylamine), poly(vinylmethylether), poly(vinylpyrrolidone), siloxane, ribonucleic acid, deoxyribonucleic acid, albumin, antibodies and their fragments, plasma proteins, collagen, elastin, fascin, fibrin, keratin, polyaspartic acid, polyglutamic acid, prolamin, transferrin, cytochrome, flavoprotein, glycoprotein, heme protein, lipoprotein, metalloprotein, phytochrome, phosphoprotein, opsin, agar, agarose, alginate, araban, arabinogalactan, carrageenan, cellulose, carboxymethyl cellulose, hydroxypropylmethylcellulose and other carbohydrate-based polymers, chitosan, dextran, dextrin, gelatin, hyaluronic acid and its derivatives, mannan, pectin, rhamnogalacturonan, starch, hydroxyalkyl starch, xylan, and its copolymers and functionalized derivatives.

Preferably, the hydrogel is a hydrogel based on biodegradable polyethylene glycol (PEG).

Preferably, the polyethylene glycol (PEG) is a group formed by mixing ethylene glycol with single molecular weight or mixing ethylene glycol with different molecular weights in a certain proportion.

The hydrogel is a shaped article, preferably in the form of microparticles. More preferably, the hydrogel is in the shape of microparticle beads. Even more preferably, the microparticle beads have a diameter of 1-1000 µm, more preferably 5-500 µm, more preferably 10-100 µm, even more preferably 20-80 µm. Bead diameter is measured while microparticle beads are suspended in an isotonic aqueous buffer. In a preferred embodiment, the hydrogel connected vinpocetine or prodrugs thereof is in the form of beads. More preferably, the hydrogel connected vinpocetine or prodrugs thereof is in the form of microparticle beads. Even more preferably, the microparticle beads have a diameter of 1-1000 µm, more preferably 5-500 µm, more preferably 10-100 µm, even more preferably 20-80 µm. Bead diameter is measured while microparticle beads are suspended in an isotonic aqueous buffer. Such hydrogels can be polymerized in different ways, for example by free radical polymerization, ionic polymerization or complex formation reactions.

If the hydrogel is processed by free radical polymerization or ionic polymerization, the at least two starting materials are a cross-linking macromer or cross-linking monomer (which is called a cross-linker reagent) and a multifunctional macromer (which is called a backbone reagent). The cross-linker reagent carries at least two functional groups that can be linked to each other, and the backbone reagent carries at least one functional group that can be linked to each other and at least one chemical functional group that is not used to participate in the polymerization step. Additional diluent monomers may or may not be present. Useful functional groups that can be linked to each other include, but are not limited to, free radical polymerizable groups such as vinyl, vinyl-benzene, acrylate, acrylamide, methacrylate, methacrylamide, and ionically polymerizable groups such as oxetane, azetidine and ethylene oxide. In an alternative preparation method, the hydrogel is produced by a chemical complex-forming reaction. In this type of reaction, the starting material is at least one macromolecular starting material that has complementary functionality to carry out reactions such as condensation or addition reactions. In one embodiment, only one macromolecular starting material is used, which is a heteromultifunctional backbone reagent containing a large number of polymerizable functional groups which can be the same or different.

In addition to active ingredients such as vinpocetine, the local ocular, otic and nasal preparations of the present application may also comprise excipients. Excipients commonly used in such preparations include, but are not limited to, isotonic agents, preservatives, chelating agents, buffers, and surfactants. Other excipients include solubilizers, stabilizers, comfort enhancers, polymers, moderator, pH regulators and/or lubricants. Any of a variety of excipients may be used in the preparations of the present application, including water, mixtures of water and water-miscible solvents (such as C1-C7 alkanols), vegetable oil or mineral oil containing 0.5% to 5% nontoxic water-soluble polymer, natural products (such as alginate, pectin, tragacanth, karaya gum, guar gum, xanthan gum, carrageenan, agar and gum arabic), starch derivatives (such as starch acetate and hydroxypropyl starch) as well as other synthetic products (such as polyvinyl alcohol, polyvinylpyrrolidone, polyvinyl methyl ether, polyethylene oxide, preferably cross-linked polyacrylic acid and mixtures of these products). The concentration of excipients is typically 1 to 100,000 times the concentration of the active ingredient. In preferred embodiments, excipients to be included in the preparation are generally selected based on their inertness toward the active ingredient composition of the preparation.

For ocular, otic, and nasal preparations, suitable isoosmotic adjusting agents include, but are not limited to, mannitol, dextrose, sodium chloride, glycerin, sorbitol, and the like. Suitable buffers include, but are not limited to, phosphates, citrates, borates, acetates, and the like. Suitable surfactants include, but are not limited to, ionic and nonionic surfactants (although the nonionic surfactants are preferred polysorbate 80, RLM 100, POE 20 cetylstearyl ether (such as CS20) and poloxamer (such as F68). The preparations may contain substances that increase the viscosity of the solution or suspension, such as sodium carboxymethylcellulose, hypromellose, microcrystalline cellulose, sorbitol or dextran. Optionally, the preparations may also contain suitable stabilizers or regents that increase the solubility of the compounds to enable the formulation of highly concentrated solutions, including, but not limited to, ethanol, benzyl alcohol, polyethylene glycol, phenethyl alcohol, and glycerol.

The preparations given herein may contain one or more preservatives. Examples of such preservatives include benzalkonium chloride, parabens, sodium perborate, sodium chlorite, alcohols (such as chlorobutanol, benzyl alcohol or phenethyl alcohol), guanidine derivatives (such as polyhexamethylene biguanidine), sodium perborate, polyquaternium-1, aminoalcohols (such as AMP-95) or sorbic acid. In certain embodiments, the preparations themselves may be preservative such that a preservative is not required. In some embodiments, although the preparations themselves is not preservative, packaging materials or packaging designs can be used to avoid drug deterioration (such as changes in physical and chemical properties and/or changes in biological effects), thereby eliminating the need for preservatives.

For ocular, otic or nasal administration, the preparation may be a solution, suspension or gel. In a preferred aspect, the preparations in aqueous solutions or suspensions for local application to the eye or ear are in the form of drops, the preparations in aqueous solutions or suspensions for local application to the nose are in the form of drops, sprays or aerosols. The term "aqueous" generally refers to aqueous preparations in which the preparations contain > 20%, > 50%, more preferably > 75% and especially > 90% of water by weight. These drops may be delivered from a single dose eye drop bottle, which may preferably be sterile, thereby enabling the bacteriostatic components of the preparation to be unnecessary. Alternatively, the drops may be delivered from a multi-dose eye drop bottle, which may preferably include a device for withdrawing any preservative therefrom as the preparation is delivered, such devices are known in the art. Solution and suspension preparations are available for nasal administration using a nebulizer. Intranasal delivery of solutions, suspensions, or dry powders may also be facilitated by propellant-based aerosol systems, and the propellant includes but not limited to a hydrofluorane-based propellant, or the active pharmaceutical ingredients can be delivered in the form of dry powders.

For ocular conditions, the components of the present application may be delivered to the eye as a concentrated gel or similar vehicle or as a soluble insert placed under the eyelids.

In a particular embodiment, the preparations of the present application are administered once daily. However, the preparations may be formulated for administration at any frequency of administration, including once a week, once every 5 days, once every 3 days, once every 2 days, twice a day, three times a day, four times a day, five times a day , six times a day, eight times a day, hourly, or any higher frequency. This dosing frequency is also maintained for varying lengths of time depending on the treatment regimen. The duration of a particular treatment regimen can vary from a single dose to a regimen that extends over months or years. The preparations are administered in varying doses, but a typical dose is one to two drops per administration, or an equivalent amount of gel or other preparations (for example, tablets, eye ointments). Those of ordinary skill in the art are familiar with determining treatment regimens for specific indications.

Gels for local or transdermal administration may generally contain a mixture of volatile solvents, non-volatile solvents and water. In certain embodiments, the volatile solvent component of the buffer solvent system may include lower (C1-C6) alkyl alcohols, lower alkyl glycols, and lower glycol polymers. In a further embodiment, the volatile solvent is ethanol. The volatile solvent component is thought to act as a penetration enhancer while also producing a cooling effect on the skin as it evaporates. The non-volatile solvent portion of the buffer solvent system is selected from lower alkylene glycols and lower glycol polymers. Propylene glycol is used in certain embodiments. The non-volatile solvent retards the evaporation of the volatile solvent and reduce the vapor pressure of the buffer solvent system. The amount of this non-volatile solvent component, like the volatile solvent, is determined by the pharmaceutical compound or drug used. When there are too few non-volatile solvents in the system, the drug compound may crystallize due to evaporation of the volatile solvent, while too much non-volatile solvents may result in a lack of bioavailability due to poor release of the drug from the solvent mixture. The buffer component of the buffer solvent system can be selected from any buffer commonly used in the art; water is used in certain embodiments. Common ingredient ratios are about 20% of non-volatile solvent, about 40% of volatile solvent and about 40% of water. Several optional ingredients can be added to local compositions. These include, but are not limited to, chelating agents and gelling agents. Suitable gelling agents may include, but are not limited to, semi-synthetic cellulose derivatives (such as hydroxypropyl methylcellulose) and synthetic polymers, galactomannan polymers (such as guar gum and its derivatives) and cosmetic agents.

Lotions include lotions suitable for application to the skin or eyes. Ophthalmic lotions may comprise sterile aqueous solutions, optionally containing a bactericidal agent, and may be prepared by methods similar to those for preparing drops. Lotions or liniments for application to the skin may also include agents to accelerate drying and cooling of the skin (such as alcohols or acetone) and/or humectants (such as glycerin) or oils (such as castor oil or peanut oil).

The pharmaceutical composition of the present application may be a cream, ointment (such as 3% ointment, ointment) or paste, which is a semi-solid preparation of the active ingredient for external application. They can be prepared by mixing the active ingredients in finely divided or powdered form alone or in a solution or suspension of an aqueous or non-aqueous fluid with a greasy or non-greasy matrix by means of a suitable machine. The matrix may include hydrocarbons such as petroleum jelly, hard paraffin, soft paraffin or liquid paraffin, glycerin, beeswax, metallic soaps; gel and slurry; oils from natural sources such as fish, almond, corn, peanut, castor or olive oil; lanolin or its derivatives or fatty acids (such as stearic acid or oleic acid) together with alcohols (such as propylene glycol) or macrogels or lanosterol or dihydrolanosterol. The preparations may incorporate any suitable surfactants, such as anionic, cationic or nonionic surfactants, such as sorbitan esters or polyoxyethylene derivatives thereof. Suspending agents such as natural gums, cellulose derivatives or inorganic materials such as siliceous silica, and other ingredients such as lanolin and lanosterol, may also be included.

Drops or sprays may contain sterile aqueous or oily solutions or suspensions, and may be prepared by dissolving the active ingredients in suitable bactericides and/or fungicides and/or any other suitable preservatives and, in certain embodiments, aqueous solutions containing surfactants. The resulting solution can then be clarified by filtration, transferred to a suitable container, which is then sealed and sterilized by autoclaving or holding at 98-100°C for half an hour. Alternatively, the solution can be sterilized by filtration and transferred to containers by aseptic technique. Examples of bactericides and fungicides suitable for inclusion in drops are azoles (for example, econazole), phenylmercuric nitrate or phenylmercuric acetate (0.002%), benzalkonium chloride (0.01%) and chlorhexidine acetate (0.01%). Suitable solvents for preparing oily solutions include glycerol, dilute alcohols and propylene glycol.

Preparations for local administration in the mouth (for example, bucally or sublingually) include troches containing the active ingredient in a flavored matrix (such as sucrose and gum arabic or tragacanth) and lozenges containing the active ingredient in a matrix such as gelatin and glycerol or sucrose and gum arabic.

For administration by inhalation, compounds such as vinpocetine mentioned in this patent may be conveniently delivered from an insufflator, a nebulizer pressurized pack, or other convenient device for delivering an aerosol spray. The pressurized package may contain a suitable propellant such as hydrofluorocarbon, dichlorodifluoromethane, trichlorofluoromethane, dichlorotetrafluoroethane, carbon dioxide or other suitable gases. In the case of pressurized aerosols, the dosage unit may be determined by providing a valve to deliver a metered amount. Alternatively, for administration by inhalation or insufflation, the compound according to the present application may take the form of a dry powder composition, for example a powder mixture of the compound with a suitable powder matrix such as lactose or starch. The powder composition may be provided in unit dosage form, for example in capsules, cartridges, gelatin or blister packs, from which the powder may be administered by means of an inhaler or insufflator.

Preferred unit dosage preparations are those containing an effective dose or an appropriate fraction thereof of the active ingredient as described herein.

It would be understood that, in addition to the ingredients specifically mentioned above, the above preparations may further include other agents conventional in the art for the type of preparation of interest, such as microinjections, preparations suitable for oral or intranasal administration (which may include flavoring agents).

The compounds may be administered orally or via injection at a dose of 0.001 to 300 mg/kg per day. The dosage range for adults and the elderly is generally 5 mg to 100 mg/day, while the dosage for children or adolescents should be reduced or maintained unchanged. Tablets or other presentation forms provided in discrete units may conveniently contain an amount of one or more compounds which is effective at such dose or doses, for example containing units of 0.1 mg to 1000 mg, usually about 1 mg to 10 mg, preferably 5 mg.

Compounds such as vinpocetine mentioned in this patent may be administered in various ways, such as oral administration, local administration or by injection, or systemic administration and local administration at the same time, or systemic administration and local administration alternately. The precise amount of the compound administered to the patient is the responsibility of the visiting physician. The specific dosage level for any particular patient depends on a variety of factors, including the activity of the specific compound used, age, weight, general health, sex, diet, time of administration, route of administration, rate of excretion, combination of drugs, the exact condition treated, and severity of the indication or the condition being treated. Additionally, the route of administration can vary depending on the condition and its severity.

In certain cases, it may be appropriate to administer at least one compound described herein (or a pharmaceutically acceptable salt, ester, or prodrug thereof) in combination with another therapeutic agent. By way of example only, if one of the side effects experienced by a patient upon receiving a compound herein is hypotension, it may be appropriate to administer an anti-hypotension agent in combination with the initial therapeutic agent. Alternatively, by way of example only, the efficacy of one of the compounds described herein may be enhanced by administration of an anti-allergic agent (*i.e*., the anti-allergic agent may have only minimal therapeutic benefit by itself, but when combined with the therapeutic agents mentioned herein, the overall treatment benefit to patients is enhanced). Alternatively, by way of example only, the benefits experienced by a patient may be enhanced by administering one of the compounds described herein with another therapeutic agent that also has a therapeutic benefit (which also includes a therapeutic regimen). By way of example only, in myopia treatment involving administration of one of the compounds described herein, the therapeutic benefit may be enhanced by further providing the patient with another therapeutic agent for myopia. In any case, regardless of the disease, disorder, or condition being treated, the total benefit experienced by the patient may be simply addition of the two therapeutic agents, or the patient may experience synergistic benefits.

Pharmaceutical compositions in dry or liquid form may be provided as single-dose or multi-dose pharmaceutical compositions.

In one embodiment of the present application, the liquid or dry pharmaceutical composition is provided in a single dose, which means that the container in which it is provided contains one pharmaceutical dose. Alternatively, the liquid or dry pharmaceutical composition is a multi-dose pharmaceutical composition, which means that the container in which it is provided contains more than one therapeutic dose, *i.e*., the multi-dose composition contains at least 2 doses. Such multi-dose compositions may be administered to different patients in need thereof, or may be administered to one patient, where after the first dose is administered the remaining doses are stored until needed.

In another aspect of the application, the pharmaceutical composition is in a container. Containers for liquid or dry pharmaceutical compositions are, for example, eye drop bottles, syringes, vials, vials with stoppers and seals, ampoules and cartridges. In particular, liquid or dry pharmaceutical compositions are provided in syringes. If the pharmaceutical composition is a dry pharmaceutical composition, the container is preferably a dual chamber syringe. In this embodiment, the dry pharmaceutical composition is provided in the first chamber of the dual chamber syringe and the reconstitution solution is provided in the second chamber of the dual chamber syringe.

The dry composition is reconstituted prior to application to a patient in need thereof. Reconstitution can be carried out in the container in which the dry composition is provided, for example in eye drop bottles, syringes, dual chamber syringes, ampoules and cartridges. Reconstitution is performed by adding a predetermined amount of the reconstitution solution to the dry composition. The reconstitution solution is a sterile liquid such as water or a buffer, which may contain other additives such as preservatives and/or antimicrobial agents such as benzyl alcohol and cresol. Preferably, the reconstitution solution is sterile water. When the dry composition is reconstituted, it is referred to as a "reconstituted pharmaceutical composition" or "reconstituted pharmaceutical composition" or "reconstituted composition".

Ocular preparations: The pharmaceutical composition of the present application can be administered in the form of an ocular preparation, and the ocular preparation of the present application contains an ophthalmologically acceptable carrier.

The amount of the active ingredient that can be combined with the carrier materials to produce a single dosage form may vary depending upon the host treated and the particular mode of administration.

As used herein, an "ophthalmically acceptable carrier" is an ophthalmologically acceptable solvent, suspension or vehicle for applying a pharmaceutical composition to the eye of a subject. The carrier can be solid or liquid. The carrier is "ophthalmically acceptable" in the sense that the carrier is suitable for administration to the eye without causing any major adverse effects.

Typically, an ophthalmologically acceptable carrier is or includes water. Typically, the ocular preparations are in the form of eye drops or gels for application to the eye. Typically, the majority of the preparation is water. Typically, the preparation includes greater than 50 wt% (for example, greater than 60 wt%, 65 wt%, 70 wt%, 75 wt%, 80 wt%, 85 wt% or 90 wt%), more typically greater than 95 wt% (for example, 96 wt%, 97 wt%, 98 wt% or 99 wt%) of water.

In some embodiments, the ophthalmologically acceptable carrier is an oil-in-water emulsion or oil. In such embodiments, the ocular preparations may be in the form of creams for application to the eye. In such embodiments, the preparation may contain greater than 10 wt%, more typically greater than 20 wt%, of oily ingredients.

In other embodiments, the carrier can be a biodegradable polymer, such as a biodegradable polymer eye implant for sustained release of the compounds of the present application and optionally other compounds. Such as biodegradable, biocompatible polymer matrices. In one embodiment, the compounds can be embedded into a polymer matrix while maintaining structural integrity. The polymer may be natural, such as a polypeptide, protein or polysaccharide; alternatively, it can be synthetic, such as poly-alpha-hydroxy acid. Examples include carriers made of, for example: collagen, fibronectin, elastin, cellulose acetate, nitrocellulose, methylcellulose, polysaccharides, fibrin, gelatin, and a combination thereof. In one embodiment, the polymer is polylactic acid (PLA) or poly lactic-co-glycolic acid (PGLA). Polymer matrices can be prepared and isolated in a variety of forms and sizes, including microspheres and nanospheres.

In some embodiments, therapeutic compounds are prepared with carriers that would protect the therapeutic compounds against rapid elimination from the body, the carriers are for example controlled release agents, including implants and microencapsulated delivery systems. Biodegradable, biocompatible polymers can be used, such as ethylene vinyl acetate, polyanhydrides, polyglycolic acid, collagen, polyorthoesters, and polylactic acid. Such preparations can be prepared using known techniques.

Excipients: Excipients suitable for use in the ocular preparations of the present application include, for example, moderators, softeners, hypertonic agents, preservatives, buffers or pH regulators. Examples of suitable excipients include:
A. Moderators: synthetic high molecular weight cross-linked acrylic polymers (such as carbomer 974 and carbomer 980); cellulose derivatives (such as hydroxypropyl methylcellulose ("HPMC" or "hypromellose"), hydroxyethyl cellulose, methylcellulose, carboxymethylcellulose (carboxymethylcellulose)) or sodium carboxymethylcellulose (sodium carboxymethylcellulose)); dextran (such as dextran 70); gelatin; polyols (such as glycerin, polyethylene glycol 300, polyethylene glycol 400, polysorbate 80, and propylene glycol); polyvinyl alcohol; povidone (polyvinylpyrrolidone); poloxamer; and hyaluronic acid (disaccharide polymer) or a sodium or potassium salt thereof.
B. Softeners: lanolin (such as anhydrous lanolin); oily ingredients (such as light mineral oil, mineral oil, paraffin, petroleum jelly, liquid paraffin, white ointment, white petroleum jelly, white wax and yellow wax); and fish oil and castor oil.
C. Preservatives: benzalkonium chloride; sodium perborate; Oxyd (sodium chlorite 0.05%, hydrogen peroxide 0.01%); polyquaternium-1 (polymer of ethanol, 2,2',2"-nitrilotri- and 1,4-dichloro-2-butene and N,N,N',N'-tetramethyl-2-butene-1,4-diamine); sodium silver chloride; hexamethylene biguanide; oxyborate; and (sodium chlorite 0.005% m/v).
D. Ocular hypertonic agent: sodium chloride.
E. The ocular preparations of the present application may further contain preservatives that inhibit the growth of microorganisms and extend the shelf life of the preparations. Preservatives that can be used in the ocular preparations of the present application include, for example, benzalkonium chloride, sodium perborate, Oxyd (sodium chlorite 0.05%, hydrogen peroxide 0.01%), polyquaternium-1 (polymer of ethanol, 2,2',2"-nitrilotri- and 1,4-dichloro-2-butene and N,N,N',N'-tetramethyl-2-butene-1,4-diamine), sodium silver chloride, hexamethylene biguanide and oxyborate. Sodium chlorite (0.005% m/v) is a microbicide with broad spectrum antimicrobial activity and very low toxicity to mammalian cells, which protects the preparations during storage but eventually dissociates into water, sodium ions, chloride ions and oxygen after exposure to light. Because they are also found in natural tears, the risk of preservative-induced eye irritation and corneal damage is minimized. Sodium chlorite is safe and effective in the long term. This preservative has no side effects on epithelial cells *in vitro* or *in vivo* and is less disruptive to cell integrity than many other preservatives in use.

The ocular preparations of the present application may be prepared by any suitable method for preparing ocular preparations. The ocular preparations are generally sterile, and thus the method may include the step of sterilizing the ocular preparations. Preferably, the ocular preparations are transparent and have reflectivity similar to tears, a suitable pH (usually buffered to around pH 7.5) to avoid severe corneal irritation and to protect against microorganisms. The preparations of the present application suitable for local administration to the eye are preferably isotonic or slightly hypotonic in order to counteract any tear hypertonicity caused by evaporation and/or diseases. This may require a tonicity agent to bring the osmolality of the preparations to or near a level of 210-320 milliosmole/kg (mOsm/kg). The preparations of the present application generally have an osmolality in the range of 220-320 mOsm/kg, and preferably have an osmolarity in the range of 235-300 mOsm/kg. Surface tension values that are close to or lower than those observed for tear films are generally preferred. The ocular preparations are generally formulated as sterile aqueous solutions.

In certain ocular embodiments, the compositions of the present application are formulated with one or more tear substitutes. Various tear substitutes are known in the art, including but not limited to: monomeric polyols such as glycerin, propylene glycol and ethylene glycol; polymeric polyols, such as polyethylene glycol; cellulose esters such as hydroxypropylmethylcellulose, sodium carboxymethylcellulose, and hydroxypropylcellulose; dextran, such as dextran 70; vinyl polymers such as polyvinyl alcohol; and carbomers, such as carbomer 934P, carbomer 941, carbomer 940, and carbomer 974P. The preparations or pharmaceutical compositions of the present application can be used together with contact lenses, corneal contact lens or other ophthalmic products (such as framed glasses, myopia treatment devices).

Preferred preparations are prepared using a buffer system that maintains the pH of the preparations between a pH of about 4.5 and a pH of about 8. Most preferred preparations have a pH of 5.5 to 7.5. Optional buffers are physiologically tolerated buffers that maintain the pH in the desired range, such as sodium phosphate, bicarbonate, succinate, histidine, citrate and acetate, sulphate, nitrate, chloride and pyruvate. Antacids such as Mg(OH)₂ or ZnCO₃ may be also used. Buffering capacity may be adjusted to match the conditions most sensitive to pH stability.

The ocular preparations of the present application may also contain cooling agents, such as menthol, camphor, borneol, geraniol, cineole, linalool, *etc.* When mixed with a cooling agent, the concentration of the cooling agent is preferably 0.0001 to 0.1 w/v%.

The ocular preparations of the present application may also comprise other commonly used therapeutic components in ophthalmology, such as bendazac and salt forms thereof (such as bendazac lysine )., decongestants (e.g., epinephrine, epinephrine hydrochloride, ephedrine hydrochloride, tetrahydrozoline hydrochloride, naphazoline hydrochloride, naphazoline nitrate, phenylephrine hydrochloride, dl-methylephedrine hydrochloride, etc.), anti-inflammatory agent/astringent (e.g., neostigmine methyl sulfate, ε-aminocaproic acid, allantoin, berberine chloride hydrate, berberine sulfate hydrate, sodium gualenate, dipotassium glycyrrhizinate, zinc sulfate, zinc lactate, lysozyme hydrochloride, etc.), antihistamines (such as diphenhydramine hydrochloride, chlorpheniramine maleate, etc.), water-soluble vitamins (sodium flavine adenine dinucleotide, cyanocobalamin, pyridoxine hydrochloride, panthenol, calcium pantothenate, sodium pantothenate, etc.), amino acids (such as potassium L-aspartate, magnesium L-aspartate, potassium L-aspartate and magnesium L-aspartate (mixture of equal amounts of both), aminoethyl sulfonic acid, etc.), ingredients that relieve visual fatigue and/or dry eyes (such as taurine, vitamin A, hypromellose and vitamin E), polyunsaturated fatty acids (such as fish oil, omega-3 unsaturated fatty acids, DHA, EPA), piracetam, sulfonamides, niacin, retinoic acid and lutein, salidroside, formononetin, atropine, dibazole, M receptor blockers (such as blockers or antagonists or inhibitors targeting M3 receptors), *etc.*

The active substance content of the pharmaceutical composition of the present application: When the drugs are mixed, the effective suitable amount of each substance can be selected for the concentration of the therapeutically active compound, and the therapeutically active compound (such as vinpocetine) in the pharmaceutical composition can exist in high concentration, medium concentration or low concentration from the perspectives of eye irritation, preparation stability and the like. For example, the therapeutically active compound accounts for about 0.0000001-100%, about 0.00001-10%, about 0.001-20%, about 0.1-30%, about 1-40%, about 10-50%, about 20-60%, about 30-70%, about 40-80%, about 50-90% (w/v) of the total amount of the pharmaceutical composition, for example, about 1 µM, about 0.1 µM, about 0.01 µM, about 5 µM, about 10 µM, about 15 µM, about 25 µM, about 50 µM; or the therapeutically active compound accounts for about 0.0000001%, about 0.000001%, about 0.00001%, about 0.0001%, about 0.001%, about 0.01%, about 0.1%, about 1%, about 10% (w/v) of the total amount of the pharmaceutical composition.

Delivery of ocular preparations: The ocular preparations of the present application may be delivered to the patient as eye drops (in the form of single-dose or multi-dose droppers), ointments, gels, creams, or biodegradable polymeric eye implants (designed for sustained release) or via ocular moistening (for example, multi-dose spray).

Packaging of the ocular preparations should relate to the retention or non-retention nature of the solution. Packaging methods such as forming-filling-packaging technology, which combines blow molding, aseptic filling and sealing into a single process, may be particularly useful for packaging preservative-free preparations in unit dose containers. Typically, these single-dose containers are composed of low-density polyethylene or polypropylene and include unscrewing lids.

The preparations and methods of the present application are already being used in any subject that could benefit from the preparations and methods of the present application. The subject is usually a mammal, more usually a human. However, the present application is not limited to treating humans and may be adapted for veterinary use. Thus, according to this application, the terms "subject", "patient" or "subject in need" include humans as well as non-human animals, including, for example, farm animals such as sheep, pigs, cattle and horses; pet animals such as dogs and cats; laboratory animals such as mice, rats, and rabbits. In a preferred embodiment, the mammal is a human.

In another aspect, the present application provides an ophthalmic device comprising a pharmaceutical composition comprising vinpocetine or a therapeutically acceptable salt thereof or a derivative thereof or a combination thereof, preferably, wherein the ophthalmic device delivers the pharmaceutical composition in a sustained release manner.

In certain embodiments of the pharmaceutical composition, ophthalmic device, or treatment method disclosed herein, the pharmaceutical composition is a sustained release preparation or subconjunctival depot.

In certain embodiments of the pharmaceutical composition, ophthalmic device, or treatment method disclosed herein, the pharmaceutical composition is a sustained release preparation comprised in an ophthalmic device.

In certain embodiments of the pharmaceutical composition, ophthalmic device, or treatment method disclosed herein, the ophthalmic device is a contact lens, a corneal contact lens, an ocular insert, a corneal covering, a corneal inlay, a nanodisc, a liposome, a nanoparticle, a punctal plug, or a hydrogel matrix with a microfluidic depot.

In certain embodiments of the pharmaceutical composition, ophthalmic device, or treatment method disclosed herein, the ophthalmic device delivers the pharmaceutical composition in a sustained release manner.

In certain embodiments of the pharmaceutical composition, ophthalmic device, or treatment method disclosed herein, the pharmaceutical composition is formulated as an ophthalmic composition, for example, as an ophthalmic composition for treating an ophthalmic disorder or condition.

In certain embodiments of the pharmaceutical composition, ophthalmic device, or treatment method disclosed herein, the pharmaceutical composition is formulated as an ophthalmic composition for treating pre-myopia, myopia, or myopia progression.

In certain embodiments of the pharmaceutical composition, ophthalmic device, or treatment method disclosed herein, the pharmaceutical composition is formulated as an ophthalmic composition for treating high myopia, moderate myopia, or low myopia.

In certain embodiments of the pharmaceutical composition, ophthalmic device, or treatment method disclosed herein, the pharmaceutical composition is formulated as an ophthalmic composition for treating patients diagnosed with pre-myopia (or at risk of developing myopia).

In certain embodiments of the pharmaceutical composition, ophthalmic device, or treatment method disclosed herein, the pharmaceutical composition is substantially uniformly distributed throughout the ophthalmic device.

In certain embodiments of the pharmaceutical composition, ophthalmic device, or treatment method disclosed herein, the ophthalmic device is comprised in a contact lens or corneal contact lens blister package.

In certain embodiments of the pharmaceutical composition, ophthalmic device, or treatment method disclosed herein, the pharmaceutical composition submerges the ophthalmic device in a contact lens or corneal contact lens blister package.

In certain embodiments of the pharmaceutical composition, ophthalmic device, or treatment method disclosed herein, the pharmaceutical composition is administered to the eye of the patient.

In certain embodiments of the pharmaceutical composition, ophthalmic device, or treatment method disclosed herein, the pharmaceutical composition is administered locally.

In certain embodiments of the pharmaceutical composition, ophthalmic device, or treatment method disclosed herein, the pharmaceutical composition is administered to the eye in the form of an eye drop preparation, an eye spray preparation, or an eye gel preparation.

In certain embodiments of the pharmaceutical composition, ophthalmic device, or treatment method disclosed herein, the pharmaceutical composition is administered to the eye in the form of an ophthalmic emulsion, an ophthalmic liposome, a nanodisc, a nanoparticle suspension or an ophthalmic ointment.

In certain embodiments of the pharmaceutical composition, ophthalmic device, or treatment method disclosed herein, the pharmaceutical composition is ophthalmically administered to the eye of a patient via the ophthalmic device.

In certain embodiments of the pharmaceutical composition, ophthalmic device, or treatment method disclosed herein, the pharmaceutical composition is administered 1, 2, 3, 4, or 5 times per day.

In certain embodiments, the pharmaceutical composition disclosed herein can be an ophthalmic aqueous preparation, such as in the form of eye drops. For example, the ophthalmic aqueous preparation described herein can be packaged in an eye drop bottle and administered as drops. In certain embodiments, the ophthalmic aqueous preparation can be administered as a single administration (*i*.*e*., a single dose), which can include one, two, three, or more drops dropped into the eye of a patient. In certain embodiments, one dose of the ophthalmic aqueous preparation described herein is one drop of the aqueous composition from the eye drop bottle.

In certain embodiments, the pharmaceutical composition disclosed herein can be an ophthalmic gel preparation. For example, the ophthalmic gel preparation can be packaged in an eye drop bottle and administered as drops. In certain embodiments, the ophthalmic gel preparation can be administered as a single administration (*i*.*e*., a single dose), which can include one, two, three, or more drops dropped into the eye of a patient. In certain embodiments, one dose of the ophthalmic gel described herein is one drop of the gel composition from the eye drop bottle.

In certain embodiments, the pharmaceutical composition disclosed herein can be an ophthalmic ointment preparation. For example, the ophthalmic ointment preparation may be packaged in a tube or other squeezable container having a dispensing spout through which the ointment strip would be delivered. In certain embodiments, the ophthalmic ointment preparation can be administered as a single administration (*i*.*e*., a single dose), which can include one or more strips into the eye of a patient. In certain embodiments, a dose of the ophthalmic ointment preparation is a strip of ointment composition dispensed through the mouth of a dispensing tube.

Treating a subject with a therapeutically effective amount of a therapeutic composition or preparation described herein may involve a single treatment or a series of treatments.

In certain embodiments of the pharmaceutical composition, ophthalmic device, or treatment method disclosed herein, the method prevents the progression of myopia in the patient being treated.

In certain embodiments of the pharmaceutical composition, ophthalmic device, or treatment method disclosed herein, the method controls the progression of myopia in the patient being treated.

In certain embodiments of the pharmaceutical composition, ophthalmic device, or treatment method disclosed herein, the method alleviates the progression of myopia in the patient being treated.

In certain embodiments of the pharmaceutical composition, ophthalmic device, or treatment method disclosed herein, the method slows down or reduces the progression of myopia in the patient being treated.

In certain embodiments of the pharmaceutical composition, ophthalmic device, or treatment method disclosed herein, the method controls, slows down, reduces, delays and/or alleviates the progression of myopia of the treated patient to be within the following ranges relative to untreated patients: between about 5-95%, between about 5-90%, between about 5-80%, between about 5-70%, between about 5-60%, between about 5-50%, between about 5-40%, between about 5-30%, between about 5-20%, between about 10-100%, between about 20-90%, between about 30-90%, between about 40-90% between about 50-90%, or between about 75-90%.

In certain embodiments of the pharmaceutical composition, preparation, ophthalmic device, or treatment method disclosed herein, the use of the pharmaceutical composition, preparation, ophthalmic device or treatment method limits the amplitude of the refraction change towards negative of the eye of the administered subject to about 1.0-6.0D, 1.0-5.0D, 1.0-4.0D, 1.0-3.0D, 1.0- 2.0D, less than 6.0D, less than 5.0D, less than 4.0D, less than 3.0D, less than 2.0D and less than 1.0D.

In certain embodiments of the pharmaceutical composition, ophthalmic device, or treatment method disclosed herein, the method reverses the progression of myopia in the treated patient.

In certain embodiments of the pharmaceutical composition, ophthalmic device, or treatment method disclosed herein, the patient has high myopia, moderate myopia, or low myopia.

In certain embodiments of the pharmaceutical composition, ophthalmic device, or treatment method disclosed herein, the patient has pre-myopia (or is at risk of developing myopia).

In certain embodiments of the pharmaceutical composition, ophthalmic device, or treatment method disclosed herein, the method prevents, controls, slows down, reduces, alleviates, delays and/or reverses axial (or longitudinal) growth, *i.e.*, the axial length elongation and/or the vitreous chamber length increase, of the eye of the patient being treated.

In certain embodiments of the pharmaceutical composition, ophthalmic device, or treatment method disclosed herein, the pharmaceutical composition, ophthalmic device or treatment method controls, slows down, alleviates, reduces, delays and/or reverses the axial length elongation and/or the vitreous chamber length increase of the treated patient (such as mild myopia, myopia of children or adolescents) to be within the following range relative to untreated patients: between about 5-95%, between about 5-90%, between about 5-80%, between about 5-70%, between about 5-60%, between about 5-50%, between about 5-40%, between about 5-30%, between about 5-20%, between about 10-100%, between about 20-90%, between about 30-90%, between about 40-90% between about 50-90%, or between about 75-90%.

In certain embodiments of the drug, preparation, composition, ophthalmic device, or treatment method disclosed herein, the method controls, terminates, slows down, reduces, delays and/or alleviates the occurrence and progression of myopia in patients diagnosed with myopia or at risk of developing myopia; increases the choroidal thickness of the eye of the patient (for example, myopic eye, pre-myopic eye or eyes at risk of developing myopia) and/or reduces the axial (or longitudinal) growth speed of the eye of the patient (for example, myopic eye, pre-myopic eye or eyes at risk of developing myopia), that is, the axial length elongation and/or the vitreous chamber length increase are inhibited.

In certain embodiments of the pharmaceutical composition, ophthalmic device, or treatment method disclosed herein, the method controls, slows down, reduces, delays and/or alleviates the axial (or longitudinal) growth of the eye of the treated patient by between about 5-95%, between about 5-90%, between about 5-80%, between about 5-70%, between about 5-60%, between about 5-50%, between about 5-40%, between about 5-30%, between about 5-20%, between about 10-100%, between about 20-90%, between about 30-90%, between about 40-90%, between about 50-90% or between about 75-90%.

In certain embodiments of the pharmaceutical composition, ophthalmic device, or treatment method disclosed herein, the pharmaceutical composition, ophthalmic device, or treatment method prevents, controls, slows down, reduces, alleviates, changes, delays and/or reverses the refraction of myopia of the eye of the treated patient.

In certain embodiments of the pharmaceutical composition, ophthalmic device, or treatment method disclosed herein, the pharmaceutical composition, ophthalmic device, or treatment method result in less severe adverse effects relative to atropine monotherapy.

In certain embodiments of the pharmaceutical composition, ophthalmic device, or treatment method disclosed herein, the pharmaceutical composition, ophthalmic device, or treatment method produces a smaller increase in pupil size or no effect on pupil size relative to atropine monotherapy.

In certain embodiments of the pharmaceutical composition, ophthalmic device, or treatment method disclosed herein, the pharmaceutical composition, ophthalmic device, or treatment method inhibits axial length or refraction progression of a normal eye toward myopia relative to atropine monotherapy.

### Example 1. Myopia animal model and administration experiment

The form deprivation and lens-induced guinea pig myopia model is a classic and recognized myopia animal model in the field. The model can be used to evaluate the efficacy and safety of drugs for treating myopia. The construction method of the model is well known to those skilled in the art. The animal modeling and administration route in this application refer to the scientific literature previously published by this research group (Sen Zhang, Invest Ophthalmol Vis Sci. 2019 Jul 1;60(8):3074-3083.; Miaozhen Pan, Exp Eye Res. 2021 Jan;202:108332.), specifically, this application uses the following exemplary construction method of the form deprivation and lens-induced guinea pig myopia model:
three-week-old tricolor guinea pigs (both male and female, simulating humans aged 3-26 years old) were used and raised in the experimental animal room of Wenzhou Medical University in a 12-hour light (400-500lux)/12-hour dark environment with free access to water and food. The myopia model adopted monocular form deprivation (FD) and lens induction (LI) modeling methods. For form deprivation, a special eye mask with a light transmittance of 1% that does not fall off on its own was used to completely cover the animal's right eye, and the other eye (left eye) obtained normal vision. In the lens induction group, a -4D lens was fixed in front of the right eye of the animal, and the left eye obtained normal vision. For the above animals, the lenses were cleaned twice a day to prevent the lenses from blurring. The animals in the experimental group were administrated at 9:00-9:30 am every day. When administration, the animals in the negative control group, the positive control group, and the experimental group respectively had the eye masks or lenses removed under red light, and were injected with 0.1 mL of solvent control, or the test drug (vinpocetine at different concentrations), or positive control (0.1% atropine) around the eyeball of the right eye. After the injection, it was confirmed that the animals were successfully administered without trauma and the eye masks or lense were immediately restored. The administration process for each animal was controlled in about 10 seconds. Administration began on the day of modeling, and was continuous for 2 weeks for the form deprivation group and was continuous for 1 week for the lens induction group. This experiment has been reviewed by the Experimental Animal Ethics Committee of Wenzhou Medical University.

The formulation method of vinpocetine eye drops used in the experimental group was as follows:
It was reported that the solubility of vinpocetine (VPN) in water at room temperature is 2.4 µg/mL. The inventors prepared a 5 µM (*i.e.*, 1.75 µg/mL) vinpocetine stock solution and stored at -80°C. The solvent was physiological saline. Before administration experiment, the stock solution was diluted with physiological saline to 1 µM, 0.1 µM, and 0.01 µM, respectively. During the formulation process of vinpocetine eye drops, the solubility can be increased by using conventional physical methods such as heating and stirring, or acids can also be used to adjust the PH value to increase solubility, and finally, the PH value can be adjusted to 7.31. The above-mentioned vinpocetine eye drops were transparent and stable at room temperature.

### Animals were grouped as follows:

### 1. Form deprivation myopia (FDM) group:

1. Form deprivation plus solvent injection group: wearing an eye mask on one eye and injecting solvent saline into the eye wearing the eye mask for 2 weeks, sample size = 20.
2. Form deprivation plus drug injection group:
   wearing an eye mask on one eye and injecting vinpocetine (0.01 µM: 0.1 mL/day) into the eye wearing the eye mask for 2 weeks, sample size = 15;
   wearing an eye mask on one eye and injecting vinpocetine (0.1 µM: 0.1 mL/day) into the eye wearing the eye mask for 2 weeks, sample size = 20;
   wearing an eye mask on one eye and injecting vinpocetine 1.00 µM: 0.1 mL/day) into the eye wearing the eye mask for 2 weeks, sample size = 22;
3. Form deprivation plus positive control group: wearing an eye mask on one eye and injecting atropine (0.1%: 0.1 mL/day) into the eye wearing the eye mask for 2 weeks, sample size = 15.

### 2. Lens induction myopia (LIM) group:

1. Lens induction plus solvent injection group: wearing a -4D lens on one eye and injecting solvent saline into the eye wearing the -4D lens for 1 weeks, sample size = 10.
2. Lens induction plus drug injection group:
   wearing a -4D lens on one eye and injecting vinpocetine (0.01 µM: 0.1 mL/day) into the eye wearing the -4D lens for 1 weeks, sample size = 12;
   wearing a -4D lens on one eye and injecting vinpocetine (0.1 µM: 0.1 mL/day) into the eye wearing the -4D lens for 1 weeks, sample size = 12;
   wearing a -4D lens on one eye and injecting vinpocetine (1.00 µM 0.1 mL/day) into the eye wearing the -4D lens for 1 weeks, sample size = 13;
3. Lens induction plus positive control group: wearing an eye mask on one eye and injecting atropine (0.1%: 0.1 mL/day) into the eye wearing the eye mask for 1 weeks, sample size = 11.

Among them, the difference between the solvent control group and the experimental groups is that the solvent control group did not contain active ingredients.

### Measurement of refractions and eyeball parameters of enrolled animals

All animals in the same model efficacy experiment were measured within the same time period, and the first measurement was at 3 weeks of age, that is, before drug administration. Refractions and eyeball parameters were measured for the animals in the above FDM group before the experiment, after 1 week of administration in the experiment and after 2 weeks of administration in the experiment and for the animals in the LIM group before the experiment, after 3 days of administration in the experiment and after 7 days of administration in the experiment, respectively. The refractions were measured using an eccentric infrared photoretinoscope (EIR) built in our laboratory. Each eye was measured three times and the average was taken as the final result. The axial parameters of the guinea pig's eyeballs were measured using the A-ultrasound probe in the CinescanA/B ultrasonic diagnostic instrument (QuantelMedical, Aviso, France). The ultrasonic frequency was 11 MHz. The ultrasonic propagation speeds of different refractive media of the eyeball were set as: 1557.5 m/s for the anterior chamber, 1723.3 m/s for the lens, and 1540 m/s for the vitreous body. The detection parameters include vitreous chamber depth (VCD) and axial length (AL). About 2 minutes before the test, 0.5% proparacaine hydrochloride eye drops (Alcon, Belgium) were used to anesthetize the ocular surface of the test eyes of the guinea pig. Each eye was measured 6 times, and the average value was taken as the final result.

### Measurement of choroidal blood flow and thickness

The choroidal blood flow parameters and choroidal thickness were measured for the animals in the above FDM group after 1 week of continuous administration and at the end of the 2 week-administration experiment and for the animals in the LIM group at the end of the 7 day-administration experiment. Choroidal blood flow parameters and thickness were measured using a modified commercial OCTA (Spectralis HRA + OCT, OCTA, Heidelberg, Germany) which is more suitable for animal eyeballs. Before the measurement, the guinea pigs were fully anesthetized (a mixture of ketamine hydrochloride 60 mg/kg and xylazine hydrochloride 9 mg/kg, intraperitoneally injected), and then the guinea pig OCTA images were obtained, and the measurement results were quantified through a self-written program (MatLab R2017a, MathWorks). Real-time quantitative detection of choroidal blood flow and thickness in guinea pigs was achieved (Sen Zhang, Invest Ophthalmol Vis Sci. 2019 Jul 1;60(8):3074-3083).

### Example 2. Experiment on the effect of local administration of vinpocetine on the eyes

The experimental method was as in example 1. In the FDM group, the refraction, vitreous chamber, and axial length of both eyes of all test animals were measured before administration, after 1 week of administration, and after 2 weeks of administration; In the LIM group, the refraction, vitreous chamber, and axial length of both eyes of all test animals were measured before administration, after 3 days of administration, and after 7 days of administration. All data were analyzed using analysis of variance of repeated measures, in which a P value less than 0.05 was considered a significant difference, and a P value less than 0.01 was considered an extremely significant difference.

### Experimental results:

1. Vinpocetine alone can effectively inhibit the occurrence and development of FDM-induced myopia (F3,73 = 14.66, p < 0.001). In the FDM group, the normal saline injection group induced myopia of -4.94 ± 2.35 D after one week of administration, and induced myopia of -6.74 ± 1.90D after two weeks of administration. The vitreous chamber depth and the axial length were correspondingly elongated, indicating that the modeling was successful. After one week of continuous administration of 1 µM vinpocetine, FDM myopia and the corresponding elongation of the axial length and vitreous chamber depth could be effectively inhibited, specifically: FDM + Vehicle vs. FDM + VPN(1 µM): -4.94 ± 2.35 vs. -2.44 ± 1.88 D, p < 0.01, the refraction inhibition rate was 50.5%; the vitreous chamber depth was also inhibited accordingly, FDM + Vehicle vs. FDM + VPN (1 µM): 0.11 ± 0.04 vs. 0.06 ± 0.05 mm, p < 0.01, the inhibition rate for the vitreous chamber depth was 45.0%, the axial length was also inhibited, FDM + Vehicle vs. FDM + VPN(1 µM): 0.11 ± 0.05 vs. 0.07 ± 0.05 mm, p = 0.09, the inhibition rate for the axial length was 39.8%. In addition, the corresponding indicators and results of myopia treatment in the administration group with medium-concentration vinpocetine also suggested that vinpocetine and its related compounds can be used for myopia treatment. The inventor even found at two time points of efficacy determination in the administration group with low-concentration vinpocetine (only 0.01 µM) that although there was no statistical difference, the process of the refraction change towards negative and the vitreous chamber depth elongation showed an inhibitory trend compared with the negative control group. Comparing with the results of myopia treatment in the positive control group, after 1 week of administration, the model-induced myopia in the 0.1% atropine group was -3.20 ± 1.73 D, the refraction inhibition rate was 35.2%, the vitreous chamber depth was 0.08 ± 0.05 mm, the inhibition rate for the vitreous chamber depth was 31.5%, the axial length was 0.08 ± 0.05, and the inhibition rate for the axial length was 32.1%. It can be seen from the above results that high-concentration vinpocetine was more effective than atropine in preventing and controlling myopia, and medium-concentration vinpocetine had similar efficacy to atropine in treating myopia (Fig. 1A-C).

Vinpocetine can also effectively inhibit myopia in the FDM model after two weeks of continuous administration. FDM + Vehicle vs. FDM + VPN(1 µM): -6.74 ± 1.90 vs. -3.36 ± 2.21 D, p < 0.001, the refraction inhibition rate was 50.1%, the refraction even had almost no change compared with the last test in some animals, the vitreous chamber depth was also inhibited accordingly, FDM + Vehicle vs. FDM + VPN (1 µM): 0.16 ± 0.07 vs. 0.09 ± 0.05 mm, p < 0.001, the inhibition rate for the vitreous chamber depth was 43.4%, the axial length was also inhibited accordingly, FDM + Vehicle vs. FDM + VPN(1 µM): 0.16 ± 0.06 vs. 0.08 ± 0.05 mm, p < 0.001, the inhibition rate for the axial length was 48.2%; At this time, the myopia induced by 0.1% atropine was -3.38 ± 2.79 D, the refraction inhibition rate was 49.8%, the vitreous chamber depth was 0.10 ± 0.05 mm, the inhibition rate for the vitreous chamber depth was 38.2%, the axial length was 0.10 ± 0.05 mm, and the inhibition rate for the axial length was 41.0% (Fig. 1A-C), pupillary dilation was observed in animals during the use of atropine.

In addition, it can be seen from Fig. 1 that vinpocetine has a positive dose-effect relationship in the treatment of form deprivation myopia model. As the dosage increases, the therapeutic effect on myopia is stronger, and likewise, vinpocetine also shows the inhibitory effect on axial length and vitreous chamber depth. Compared with the control group, vinpocetine can significantly inhibit the refraction change towards negative in form-deprived myopia, and significantly inhibit the axial length elongation and the vitreous chamber depth elongation in form-deprived myopia. At the same time, the overall performance of vinpocetine in the treatment of myopia in the FDM model is also better than that of atropine, especially in the inhibitory effect on the progression of the refraction change towards negative.

2. Vinpocetine can effectively inhibit the occurrence and development of LIM-induced myopia (F3,43 = 4.073, p = 0.012). In the LIM group, the normal saline injection group induced myopia of -3.25 ± 1.20 D after 3 days of experiment , and induced myopia of -3.93 ± 1.26 D after 7 days of experiment. The vitreous chamber depth and the axial length were correspondingly elongated or increased. After 3 days of administration, 1 µM vinpocetine could effectively inhibit myopia in the LIM model, LIM + Vehicle vs. LIM + VPN (1 µM): -3.25 ± 1.20 vs. -2.00 ± 0.82 D, p < 0.05, the refraction inhibition rate was 38.5%, the vitreous chamber depth was also inhibited accordingly, LIM + Vehicle vs. LIM + VPN (1 µM): 0.07 ± 0.03 vs. 0.03 ± 0.03 mm, p < 0.01, the inhibition rate for the vitreous chamber depth was 58.8%, the axial length was also inhibited accordingly, LIM + Vehicle vs. LIM + VPN(1 µM): 0.08 ± 0.02 vs. 0.04 ± 0.02 mm, p < 0.001, the inhibition rate for the axial length was 55.5%; At this time, the myopia induced by 0.1% atropine was -2.34 ± 0.73 D, the refraction inhibition rate was 30.0%, the vitreous chamber depth was 0.04 ± 0.03 mm, the inhibition rate for the vitreous chamber depth was 47.3%, the axial length was 0.04 ± 0.02 mm, and the inhibition rate for the axial length was 47.4%. It could be seen that all myopia treatment indicators in the high-concentration (1 µM) vinpocetine group were better than those of atropine. Compared with the negative control group, the high-, medium- and low-concentration vinpocetine administration groups all showed the effect of myopia treatment (Fig. 2A-C).

Vinpocetine could also effectively inhibit myopia in the LIM model after 7 days of administration. By comparing the change trend in the process of the refraction change towards negative in different experimental groups during the entire administration period, it was found that vinpocetine could significantly slow down the rate of the aggravation of myopia in myopia model of lens induction. The results for refraction were LIM + Vehicle vs. LIM + VPN (1 µM): - 3.93 ± 1.26 vs. -2.61 ± 1.17 D, p < 0.05, the refraction inhibition rate was 36.3%, the vitreous chamber depth was also inhibited accordingly, LIM + Vehicle vs. LIM + VPN (1 µM): 0.09 ± 0.04 vs. 0.05 ± 0.02 mm, p < 0.001, the inhibition rate for the vitreous chamber depth was 49.8%, the axial length was also inhibited accordingly, LIM + Vehicle vs. LIM + VPN(1 µM): 0.10 ± 0.04 vs. 0.05 ± 0.02 mm, p < 0.001, the inhibition rate for the axial length was 53.8%; At this time, the myopia induced by 0.1% atropine was -2.61 ± 1.17 D, the refraction inhibition rate was 33.4%, the vitreous chamber depth was 0.05 ± 0.03 mm, the inhibition rate for the vitreous chamber depth was 45.7%, the axial length was 0.06 ± 0.03 mm, and the inhibition rate for the axial length was 41.8% (Fig. 2A-C), pupillary dilation was observed in animals during the use of atropine. It could be seen that the overall performance of high-concentration vinpocetine in the treatment of myopia in the LIM model was better than (high-concentration) atropine, especially when evaluating the druggability of the two from the perspective of drug safety and effectiveness.

It could also be seen from Fig. 2 that there was a positive dose-effect relationship for different concentrations of vinpocetine in the treatment of myopia model of lens induction. At the same time, vinpocetine could have a high inhibitory effect in the early stages of myopia and could maintain this inhibitory effect. That is, the excellent efficacy of vinpocetine in preventing and treating myopia had nothing to do with the severity of myopia or the speed of the refraction change towards negative. In addition, compared with the normal saline group, all vinpocetine treatment groups significantly inhibited the progression of the refraction change towards negative of lens-induced myopia, delayed the occurrence and development of myopia, and significantly inhibited the axial length elongation and vitreous chamber depth elongation of lens-induced myopia during almost the entire administration cycle.

Based on the data of two recognized animal models for myopia efficacy evaluation, vinpocetine showed excellent therapeutic effect on myopia, that is, the degree of inhibiting and slowing down the process of the refraction change towards negative was consistent with the corresponding inhibition of axial length elongation and vitreous chamber depth increase, demonstrating that vinpocetine mainly treated myopia by controlling the axial length elongation and the vitreous chamber depth increase. The inventors unexpectedly discovered that when used alone, vinpocetine had even greater efficacy than high-grade atropine (0.1% atropine) in the treatment of myopia, especially axial myopia. It was worth noting that the above-mentioned therapeutic effect of vinpocetine on myopia had nothing to do with the gender, age, type of myopia, myopia progression rate and severity of myopia of the subjects.

No ocular abnormalitieswere observed in all test animals in the vinpocetine group during administration. No significant changes in the pupil size of the test eyes were observed during the local administration of vinpocetine to young guinea pigs, and there was no allergic phenomenon. At the same time, no individual was observed to have common clinical adverse reactions or toxic manifestations such as weight loss for vinpocetine during the administration of all test animals in the vinpocetine group.

From the perspective of pharmacodynamic result evaluation and the safety of long-term continuous medication, vinpocetine could be used to prevent, treat and control myopia, especially in children and adolescents.

### Example 3. Mechanism of vinpocetine in treating myopia

Vinpocetine is known to improve blood circulation. The inventors used the method of example 1 to detect changes of indicator signals of choroidal thickness ChT and choroidal blood flow ChBP of FDM (1 week and 2 weeks of administration) and LIM (1 week of administration) at different time points during the administration cycle. It was found that: compared with the solvent injection group, therapeutically effective amount of vinpocetine only slightly increased choroidal thickness and choroidal blood perfusion but did not increase statistically significantly (in the FDM model, ChBP: F_{3,59} = 1.294, p = 0.285, Fig. 3A-B; in the LIM model, ChBP: F_{3,44} = 0.383, p = 0.766, Fig. 4A-B). In addition, after comprehensively investigating the inhibition of the drugs on myopia treatment indicators such as refraction and axial length and the effect of vinpocetine on choroidal thickness increase and choroidal blood perfusion at corresponding time points, it was found that real efficacy of myopia treatment and myopia progression control and the assumed pharmacology of dilating blood vessels (increasing choroidal blood flow) were not logically corresponding. There were contradictions in the relevant test data of the form deprivation group after one week of administration, that is, different concentrations of vinpocetine could effectively inhibit the refraction change towards negative and the vitreous chamber depth increase, showing significant therapeutic effect on myopia and a positive dose-effect relationship; but the effect of vinpocetine on choroidal blood perfusion showed a negative dose-effect relationship, and the effect of different concentrations of vinpocetine on choroidal blood perfusion was similar to the tendency shown by negative control group compared with atropine group. In particular, the high-concentration group of vinpocetine (1 µM), although the least effective at improving choroidal blood flow, had the strongest effect on inhibiting the progression of the refraction change towards negative in myopia (Fig. 1A-B and Fig. 3B); Another example is that at 2 weeks, the medium concentration (0.1 µM) of vinpocetine in the form deprivation group showed a therapeutic effect on myopia similar to that of atropine in terms of axial parameters, but its effect on choroidal vasodilation was not as good as atropine (Fig. 1B-C and Fig. 3B).

To sum up, the pharmacological mechanism of vinpocetine in treating myopia was not by dilating ocular blood vessels, improving ocular blood circulation, or increasing choroidal blood flow, but may rely more on a mechanism that has never been identified (ocular) function, and the biological target corresponding to this function even had a competitive binding relationship with the known target of vinpocetine to dilate blood vessels.

### Example 4. Effect of vinpocetine on controlling the myopia progression through systemic administration

The purpose of this experiment was to evaluate the effect of vinpocetine on myopia treatment, especially on the control of myopia progression, through systemic administration (such as intravenous drip or gavage administration), and the effectiveness and safety of the vinpocetine were comprehensively analyzed based on indicators such as refraction, axial length, vitreous chamber depth, and body weight. In rats, the oral bioavailability of vinpocetine is 52% (Vereczkey *et al.*, 1979a), and the LD50 value by gavage administration was approximately 500 mg/kg (Cholnoky and Dömök, 1976). The literature also reported that after administering lethal doses of vinpocetine to rodents, the animals often developed clinical manifestations of ataxia and clonic convulsions (Cholnoky and Dömök, 1976). According to the rodent safety evaluation data of vinpocetine published by the FDA, when the animals of childbearing age were administrated by gavage, there was no obvious abnormality when rats were administrated at 20 mg/kg per day, but when they were administrated at 40 mg/kg or more, they showed signs of weight loss and food intake reduction; rabbits were administrated at 75 mg/kg per day without any obvious abnormalities, but began to show toxic effects of weight loss and food intake reduction at 150 mg/kg. The dosage forms of vinpocetine currently sold in China include tablets and injections. Based on the normal living habits of guinea pigs and the requirements for form deprivation modeling, as well as considering the principles of medication compliance for clinical use in future myopia treatment, the inventors believe that in evaluating the efficacy of vinpocetine in the treatment of myopia, gavage administration is similar to or better than administration by intravenous drip. The dosage of a single gavage administration of guinea pigs should be determined with reference to the oral bioavailability of vinpocetine in similar species and the corresponding toxicity dose by gavage of rodents. The dosage should basically maintain the normal physiological state of the guinea pigs and not cause severe toxic reactions such as ataxia or clonic convulsions.

Continuous daily administration was used to evaluate the inhibitory effect of vinpocetine on the myopia progression by systemic administration, and the toxicity of the dosage was observed at the same time. The form deprivation myopia animal model and the method for evaluating the efficacy of vinpocetine for myopia treatment used in this experiment were the same as other relevant embodiments (local administration) in this application. Among them, the establishment of a guinea pig model of form deprivation myopia (FDM) and the gavage administration were manipulated and performed referring to the published literature in the inventor's laboratory. (Pan Miaozhen, Proc Natl Acad Sci USA. 2021 Oct 26;118(43):e2104689118). Specifically, healthy 3-week-old tricolor guinea pigs (either male or female), after excluding individuals with obvious eye diseases or abnormalities, were tested for refraction (eccentric infrared photoretinoscope) and eye axis (A-ultrasound), and animals with refractive power of 3-8 diopter(D) and binocular anisometropia less than 2D were selected and randomly divided into the following 3 groups: form deprivation myopia (FDM) + vehicle control (Vehicle) group, FDM + low-dose vinpocetine group (VPN) and FDM + high-dose VPN group, the mean body weights of the animals in the three groups before administration were basically the same and there was no statistical difference between them. On the first day of the experiment, form deprivation (FD) myopia modeling began at 8 a.m. on the guinea pigs. The form deprivation myopia model adopted the mask method. The hood was made by the inventors using a 10-inch milky white non-toxic latex balloon. The right eye of the individual guinea pig for modeling was covered by the hood (experimental eye), and the left eye was not covered by the hood (contralateral eye). FD induction was continued during the entire vinpocetine efficacy experiment cycle by gavage administration, and the hood was only briefly removed during eye testing (such as refraction testing). After the start of the efficacy experiment, the position of the hood was checked at 8 a.m., 12 at noon, 7 p.m. and before administration, and individuals who lost their hood for more than 3 times were excluded. Starting from the day of modeling, the corresponding solvent or drug was be administered to the FDM model between 9 and 10 a.m. every day. The administration route was gavage administration. The dosage was 50 mg/kg/d (low dosage) and 100 mg/kg/d (high dosage) respectively, the actual dosage was calculated based on the average weight of animals in each group, and the gavage volume of the solvent group was 5 mL/kg/d. Administration was performed once a day for 2 weeks. The refraction and axial parameters of the test animals were measured at the beginning, 1 week and 2 weeks of the efficacy experiment of this model. All data collection and processing methods were the same as those in the literature published by the inventor's laboratory (Sen Zhang, Invest Ophthalmol Vis Sci . 2019 Jul 1;60(8):3074-3083; Pan Miaozhen, Proc Natl Acad Sci USA. 2021 Oct 26;118(43):e2104689118), the statistical basis was the difference between the experimental eye and the contralateral eye of the same subject individual, and the weight of all subject individuals was recorded during the experimental period. The preparation method of vinpocetine gavage liquid used by the inventors of this embodiment is as follows: it was reported that the solubility of vinpocetine in 0.5% methylcellulose at room temperature could reach 200 mg/mL (Waidyanatha S et al, Systemic exposure of vinpocetine in pregnant Sprague Dawley rats following repeated oral exposure: An investigation of fetal transfer, Toxicol Appl Pharmacol, 338:83-92, 15 Nov 2017). Without adding other pharmaceutical excipients or other compounds, the inventors directly and completely dissolved the vinpocetine compound powder in 0.5% methylcellulose solvent (sterile water + methylcellulose) to prepare a vinpocetine preparation (gavage liquid) at 10 mg/mL and 20 mg/mL. The overall appearance of the preparation at room temperature was clear, transparent, and uniform, with no suspended matter visible to the naked eye. The preparation process of the preparation may use conventional physical and chemical solubilization means such as heating, stirring, and pH adjustment as appropriate, and no compounds would precipitate before administration of the preparation.

The experimental results found that the changes in refraction and axial parameters of the animals in the negative control group were in line with the expectations of the myopia model, proving that the form deprivation myopia model in this experiment was successful and could be used to evaluate the efficacy of the tested drugs. As shown in Fig. 5, throughout the treatment cycle, whether judged from refraction or axial parameters, the systemic administration group of vinpocetine had a tendency to treat myopia and inhibit myopia progression compared with the solvent control group. At the same time, the weight gain of the vinpocetine group was significantly inhibited. Take the low-concentration gavage group as an example: after 1 week of administration, the body weight of the vinpocetine administration group at 50 mg/kg was significantly lower than that of the solvent group, FDM + Vehicle vs. FDM + VPN (50 mg/kg): 208.5 ± 14.9 vs. 188.0 ± 22.9 mg, p < 0.05, the mean weight decreased by 9.9%; After 2 weeks of administration, the weight gain of the vinpocetine administration group at 50 mg/kg was significantly inhibited, FDM + Vehicle vs. FDM + VPN (50 mg/kg): 229.6 ± 21.4 vs. 210.2 ± 21.9 mg, p < 0.05, the mean weight decreased by 8.4%. It could be seen that when the daily dosage reached 50 mg/kg, substantial toxicity to experimental animals was already produced and continuing to increase the dosage of vinpocetine would increase the risk of toxicity to test individuals. However, it was worth emphasizing that the inventors observed that gavage administration of vinpocetine had a definite dose-effect relationship for the treatment of myopia, especially the inhibition of myopia progression: That is, as the amount of gavage administration increased, the efficacy of vinpocetine in the treatment of myopia through systemic administration became more and more obvious. Compared with the 50 mg/kg dose, when the drug was administered by gavage at the 100 mg/kg dose, the degree of refraction change towards negative and the axial length elongation of the subject individuals were further inhibited. Illustrated by the refraction and axial parameters of the three groups of animals at the end of the treatment, where the refraction: FDM + Vehicle vs. FDM + VPN (50 mg/kg) vs. FDM + VPN (100 mg/kg): -6.59 ± 1.42 vs. -5.71 ± 2.41 vs. -5.223 ± 2.20D; vitreous chamber depth: FDM + Vehicle vs. FDM + VPN(50 mg/kg) vs. FDM + VPN(100 mg/kg): 0.12 ± 0.05 vs. 0.13 ± 0.07 vs. 0.10 ± 0.06 mm; axial length: FDM + Vehicle vs. FDM + VPN (50 mg/kg) vs. FDM + VPN (100 mg/kg): 0.14 ± 0.05 vs. 0.13 ± 0.05 vs. 0.11 ± 0.05 mm. It could be concluded from the above results that: systemic administration of vinpocetine also has the potential to inhibit myopia progression and achieve significant therapeutic effects on myopia, but compared with local administration, systemic administration should require a larger dose or a longer treatment time. In terms of safety evaluation, direct systemic administration (such as direct intravenous infusion or oral tablet) of vinpocetine for the treatment of myopia would bring a certain degree of side effects (such as weight loss or increased risk of miscarriage), so local administration is more recommended, because patients who use vinpocetine to treat myopia through oral and other systemic administration would bear additional clinical risks.

It could be seen that vinpocetine could be used for the prevention and treatment of myopia, whether administered systemically or locally. Compared with systemic administration, local administration of vinpocetine is a better choice for preventing and controlling myopia progression. It should be understood that the results of this example are not inconsistent with the conclusions of this application, but are intended to remind: when directly administration vinpocetine for the prevention, control and treatment of myopia, it is preferred to use a safer, more efficient and more convenient local administration route (preferably ocular administration), but this does not rule out the use of systemic administration routes, such as the use of specific drug targeting technology, which could effectively reduce the toxicity of systemic administration of drugs and at the same time enrich the vinpocetine drug in the eye to achieve safe, efficient and convenient systemic administration to treat or inhibit myopia.

### Example 5. Treatment of myopia with salmeterol and tiotropium

The form deprivation myopia model of guinea pigs described in the present invention was used to evaluate the efficacy. All individuals were tested for refraction and eye axis, and after removing unqualified animals, they were randomly divided into 5 groups: vinpocetine alone treatment group, salmeterol + tiotropium + vinpocetine combined treatment group, salmeterol + tiotropium combined treatment group, negative control group (solvent group) and 0.1% atropine group (positive Control group), in which salmeterol (S) and tiotropium (tiotropium, T) were used at concentrations of 1 × 10⁻⁵ M and 1 × 10⁻⁶ M respectively, the concentration of vinpocetine used was 1 µM whether administered alone or in combination. On the first day of the experiment, guinea pigs were subjected to form deprivation at 8 a.m. using a mask method, with the right eye covered (experimental eye) and the left eye uncovered (contralateral eye). Starting from the day of modeling, solvents or drugs for the corresponding groups were given to the experimental eyes at 9-10 a.m. every day by eyeball subconjunctival injection at an injection volume of 100 µL, once a day for 2 weeks. The refraction and axial parameters of the animals were detected before the start of the efficacy experiment and at the end of the experiment. All data collection and processing methods were the same as in other embodiments of the present invention. The statistical basis was the difference between the experimental eye and the contralateral eye of the same subject individual.

The experimental results showed that the changes in refraction and axial parameters of the animals in the negative control group were in line with the expectations of the myopia model, and the positive control drug atropine showed its due efficacy in the experiment. These proved that the myopia model in this experiment was successful and could be used to evaluate the efficacy of the tested drugs. After the drug intervention, there was no statistical difference between the salmeterol + tiotropium combined treatment group and the negative control group in terms of refraction or axial parameters. The specific data was, after 2 weeks of administration, refraction: FDM + Vehicle vs. FDM + S + T(salmeterol, 10⁻⁵ M + tiotropium, 10⁻⁶ M): -6.00 ± 1.61 vs. -5.09 ± 1.80 D, p > 0.05, vitreous chamber depth: FDM + Vehicle vs. FDM + S + T(salmeterol, 10⁻⁵ M + tiotropium, 10⁻⁶ M): 0.12 ± 0.05 vs. 0.12 ± 0.06 mm, p > 0.05, axial length: FDM + Vehicle vs. FDM + S + T(salmeterol, 10⁻⁵ M + tiotropium, 10⁻⁶ M): 0.13 ± 0.04 vs. 0.13 ± 0.08 mm, p > 0.05. The above results could fully prove that salmeterol and tiotropium have no therapeutic effect on myopic individuals, and they could not effectively control myopia progression. Compared with the negative control group, at the end of the two weeks of administration, the vinpocetine (1 µM) alone treatment group and the 0.1% atropine group had consistent results with myopia treatment in other embodiments of the present invention, both of which significantly inhibited the refraction change towards negative, axial length elongation, and vitreous chamber depth increase of myopic individuals (as shown in Fig. 6, there was a statistical difference between the vinpocetine (1 µM) treatment group and the solvent control group). There were no abnormalities in the eyes of the vinpocetine treatment group, but pupil dilation occurred in the atropine group. The related indicators of corneal curvature (RCC), anterior chamber depth (ACD) and lens thickness (LT) of the animals in the two groups were not affected by the drug (see Fig. 6). Therefore, while meeting the ocular safety requirements for young individuals, vinpocetine, as the sole active ingredient, main active ingredient, or direct active ingredient, could effectively prevent and control myopia, and significantly inhibit, control, and slow down myopia progression.

In addition, the therapeutic effect of the salmeterol + tiotropium + vinpocetine combined treatment group was similar to that of vinpocetine (1 µM) alone. During the entire treatment period, there was no statistical difference between the two treatments in the indicators such as the refraction, axial length and vitreous chamber depth of the guinea pig myopia model, indicating that neither salmeterol nor tiotropium showed a synergistic or promoting effect on the myopia treatment with vinpocetine. Comprehensive analysis showed that there was no significant difference in effectiveness between combined administration of salmeterol + tiotropium + vinpocetine and vinpocetine alone in the treatment of myopia, in addition, salmeterol + tiotropium treatment group had no significant efficacy on myopia, thus it was concluded that in the combined administration of salmeterol + tiotropium + vinpocetine, only the vinpocetine had the effect of treating myopia, that is, vinpocetine, as the sole active ingredient, main active ingredient or direct active ingredient, could be used for myopia prevention and control and could effectively control myopia progression. The use of vinpocetine alone to treat myopia was superior to the drug combination treatment group of this embodiment in terms of safety, effectiveness and drugability.

### Example 6. Vinpocetine could effectively prevent and control myopia under the condition of non-invasive administration (eye drop dosage form)

The purpose of this experiment was to evaluate the effect of vinpocetine on myopia through non-invasive administration. All tested drugs were formulated into eye drops. The form deprivation myopia model of guinea pigs described in the present invention was used to evaluate the efficacy. All individuals were tested for refraction and eye axis, and after removing unqualified animals, they were randomly divided into 4 groups: low-concentration vinpocetine (1 µM) group, high-concentration vinpocetine (5 µM) group, negative control group (solvent group) and 0.1% atropine group (positive control group). On the first day of the experiment, guinea pigs were subjected to form deprivation at 8 a.m. using a mask method, with the right eye covered (experimental eye) and the left eye uncovered (contralateral eye). Starting from the day of modeling, solvents or drugs for the corresponding groups were given to all experimental eyes at 9-10 a.m. and 14-15 p.m. every day by direct eye drops at the volume of 25 µL for a single eye drop administration, twice a day for 2 weeks. The refraction and axial parameters of the animals were detected before the start of the efficacy experiment and at the end of the experiment. All data collection and processing methods were the same as in other embodiments of the present invention. The statistical basis was the difference between the experimental eye and the contralateral eye of the same subject individual. The vinpocetine eye drops and atropine eye drops used in this example were prepared by the inventors using 0.9% physiological saline. No other active substances or excipients were added to the above eye drops.

The experimental results showed that the changes in refraction and axial parameters of the animals in the negative control group were in line with the expectations of the myopia model, and the positive control drug atropine showed its due efficacy in the experiment. These proved that the myopia model in this experiment was successful and could be used to evaluate the efficacy of vinpocetine (eye drops).

Through non-invasive administration, vinpocetine eye drops could effectively inhibit the occurrence and development of myopia (refraction: F_{2,42} = 9.115, p < 0.001; vitreous chamber: F_{2,42} = 6.243, p = 0.04; eye axis: F_{2,42} = 8.986, p < 0.001), and there was a dose-effect relationship, that is, the higher the dosage concentration, the better the therapeutic effect on myopia (myopic eye), and there were statistical differences in the axial parameter indicators between drug groups with different concentrations of vinpocetine in myopia treatment. Due to the modeling method, the structure of the guinea pig eye (the eyeball was relatively convex compared to humans and did not actively close the eyes) and the animal's normal blinking, the actual effective therapeutic dose of the drug obtained in each subject's eye from eye drops was lower than the amount administered to eyes by peribulbar injection under the same volume of eye drop, and the daily total volume of eye drops administered to the tested animals was also smaller than the injection volume of example 2. Therefore, in this example, regardless of the low-concentration vinpocetine eye drop group or the 0.1% atropine group, the efficacy (refraction indicator) of direct administration of eye drops on myopia was inferior to that of peribulbar injection administration on myopia. The specific results were shown in Fig. 7: continuous administration of 5 µM vinpocetine for 2 weeks in the high-concentration group could effectively inhibit the progression of FDM myopia degree and the corresponding elongation of the axial length and the vitreous chamber depth, specifically: FDM + Vehicle vs. FDM + VPN(5 µM): -6.56 ± 1.70 vs. -3.80 ± 1.16 D, p < 0.001, the refraction inhibition rate was 42.1%; the vitreous chamber depth was also inhibited accordingly, FDM + Vehicle vs. FDM + VPN (5 µM): 0.14 ± 0.05 vs. 0.09 ± 0.04 mm (actual value 0.0869 ± 0.0448 mm, rounded), p < 0.01, the inhibition rate for the vitreous chamber depth was 38.6%; the axial length was also inhibited, FDM + Vehicle vs. FDM + VPN(5 µM): 0.16 ± 0.04 vs. 0.09 ± 0.04 mm (actual value 0.0925 ± 0.0406 mm, rounded), p < 0.001, the inhibition rate for the axial length was 41.4%. The low-concentration 1 µM vinpocetine administration group was also able to effectively control myopia progression: FDM + Vehicle vs. FDM + VPN(1 µM): -6.56 ± 1.70 vs. -4.81 ± 1.72 D, p < 0.05, the refraction inhibition rate was 26.7%. Comparing with the progression of myopia in the negative control group, after 2 weeks of administration, the model-induced myopia in the 0.1% atropine eye drop group was -3.93 ± 1.75 D, the refraction inhibition rate was 40.0%, the vitreous chamber depth was 0.11 ± 0.05 mm (actual value 0.1107 ± 0.0502 mm, rounded), the inhibition rate for the vitreous chamber depth was 21.8%, the axial length was 0.11 ± 0.05 mm (actual value 0.1093 ± 0.0530 mm, rounded), and the inhibition rate for the axial length was 30.7%. Adverse reactions of pupil dilation occurred during administration in the atropine group but no obvious ocular abnormalities were found in the vinpocetine group. Regardless of myopia intervention with atropine or vinpocetine, the corneal curvature, anterior chamber, and lens-related indicators of the subject individuals were not affected (see Fig. 7). In the inventor's laboratory, for 0.01% atropine eye drops which were widely used in clinical trials at present, atropine of this concentration did not have any therapeutic effect on the guinea pig myopia model under the same administration conditions as in this example (myopia model, administration route, administration frequency, administration volume, *etc.* remained the same). 0.01% atropine had no significant intervention effect on either the refraction indicator or the axial parameter indicator. This may be related to the short time that the eye drops stayed on the surface of the eyeball after administration to animals. Therefore, from the perspective of efficacy and safety, the risk-benefit ratio of vinpocetine for the treatment of individuals with myopia was better than that of atropine (for example, using vinpocetine during the daytime would not cause photophobia similar to that caused by pupil dilation with atropine during myopia treatment), especially suitable for the treatment of myopia in children and adolescents and the prevention and control of myopia in school-age people.

This example proves: The administration of vinpocetine eye drops through eye drop administration and eyeball subconjunctival injection administration both could significantly inhibit the refraction change towards negative in myopia, significantly inhibits the axial length elongation and vitreous chamber depth increase in myopia. This administration route also has a positive dose-effect relationship, that is, the higher the dosage of vinpocetine, the more obvious the therapeutic effect on myopia would be. At the same time, in the case of non-invasive administration, the overall performance of vinpocetine in the treatment of myopia is also better than that of the positive control atropine, especially in the control effect of myopia progression.

### Example 7. Vinpocetine metabolite Apovincaminic acid (AVA) could effectively prevent and control myopia

The form deprivation myopia model of guinea pigs described in the present invention was used to evaluate the efficacy. All individuals were tested for refraction and eye axis, and after removing unqualified animals, they were randomly divided into 3 groups: apovincaminic acid alone group, negative control group (solvent group) and 0.1% atropine group (positive control group), in which the concentration of apovincaminic acid (AVA) was 1 µM. The modeling method for myopic individuals was the same as other embodiments of the present invention. All groups were administered by eyeball subconjunctival injection at an injection volume of 100 µL, once a day for one week. The refraction and axial parameters of the animals were detected before the start of the efficacy experiment and at the end of the experiment. All data collection and processing methods were the same as in other embodiments of the present invention. The statistical basis was the difference between the experimental eye and the contralateral eye of the same subject individual. The AVA injection and atropine injection used in this example were prepared by the inventors using 0.9% physiological saline. No other active substances or excipients were added.

The experimental results showed that the changes in refraction and axial parameters of the animals in the negative control group were in line with the expectations of the myopia model, and the positive control drug atropine showed its due efficacy in the experiment. These proved that the myopia model in this experiment was successful and could be used to evaluate the efficacy of AVA. Experimental results demonstrated that vinpocetine metabolite AVA could effectively inhibit the occurrence and development of FDM-induced myopia (refraction: F_{2,36} = 7.914, P = 0.001; vitreous chamber: F_{2,36} = 3.904, p = 0.029; eye axis: F_{2,36} = 4.539, p = 0.017). Continuous administration of 1 µM AVA for 1 week could effectively inhibit the progression of myopia degree and the corresponding elongation of the axial length and vitreous chamber depth (see Fig. 8), specifically: FDM + Vehicle vs. FDM + AVA(1 µM): -4.39 ± 1.73 vs. -2.65 ± 1.18 D, p < 0.01, the refraction inhibition rate was 39.6%; the vitreous chamber depth was also inhibited accordingly, FDM + Vehicle vs. FDM + AVA (1 µM): 0.09 ± 0.04 vs. 0.06 ± 0.02 mm, p < 0.05, the inhibition rate for the vitreous chamber depth was 34.7%; the axial length was also inhibited, FDM + Vehicle vs. FDM + AVA (1 µM): 0.10 ± 0.04 vs. 0.07 ± 0.03 mm, p < 0.01,the inhibition rate for the axial length was 36.4%. Pupil dilation occurred in the atropine group during administration while no ocular abnormalities were observed in the AVA group. It could be seen from the above results that vinpocetine metabolite AVA also has significant efficacy in the prevention, control and treatment of myopia.

In summary, vinpocetine could not only significantly slow down the procession of refraction change towards negative in individuals with myopia or individuals with a tendency to develop myopia, but could also significantly inhibit the vitreous chamber depth increase and the axial length elongation. Vinpocetine could significantly improve symptoms related to myopia and achieve the effects of preventing, treating and controlling myopia progression and no eye abnormality is observed in all subject individuals during the administration, and no obvious change is observed in the pupil size of the subject eyes during the administration. Therefore, vinpocetine could be used in the prevention, control and treatment of myopia to delay the occurrence and development of myopia, control the progression of myopia, especially in children and adolescents with myopia or simple axial myopia or progressive myopia or primary myopia, and treat, prevent and control or improve these related symptoms of myopia.

## Claims

1. Use of vinpocetine, or an optical isomer or a racemate, or a solvate, or a pharmaceutically acceptable salt, or a prodrug, or a metabolite, or a related compound or extract, or a crystalline compound thereof, or a combination of these substances, wherein, the use is one or two or more selected from the following:
① preventing and/or treating myopia and related symptoms thereof, and/or myopia correction; or
② inhibiting (controlling) the progression of myopia; or
③ preventing and controlling or delaying the occurrence and development of myopia (myopic eye); or
④ inhibiting and slowing down axial length elongation and/or vitreous chamber length (depth) increase in an individual with myopia or an individual with a tendency to develop myopia; or
⑤ preventing, slowing down, attenuating or treating the abnormal development of eyeballs associated with visual impairment; or
⑥ allowing an individual to obtain clearer distance vision without wearing glasses (such as framed glasses or OK lenses) or relying on vision correction means (such as refractive surgery) than the distance vision before using these substances or means and/or than the distance vision without using these substances or means; or
⑦ inhibiting, delaying or slowing down the procession or speed of the refraction change towards negative in an individual with myopia or an individual with a tendency to develop myopia; or
⑧ preparing a drug, a preparation, a composition or a device, which are used to achieve at least one of ①-⑦ as stated above.

2. The use of claim 1, wherein systemic administration (such as oral administration, intravenous drip), and/or local administration (eye drops, eye injection, eye implant, skin ointment/periorbital emulsion application or eye ointment application), and/or parenteral administration (such as mucosal administration, transdermal administration, microneedle administration), and/or non-invasive administration (such as administration via eye spray) is adopted.

3. The use of claim 1 or 2, wherein the drug, preparation or composition can be an injection, a tablet, a freeze-dried powder for injection, a capsule, an effervescent tablet, a chewable tablet, a buccal tablet, a granule, an ointment, a syrup, an oral liquid, an aerosol, a nasal drop, a topical preparation, an oral preparation, *etc*.; preferred is an ocular dosage form, including but not limited to an eye drop (eyewash), an eye ointment, an eye spray, an implant tablet, an eye gel, an eye patch, an eye microsphere, an eye sustained-release preparation, a periorbital injection, and an intraocular injection; the drug, preparation or composition can also be a free solution, an oil-water mixture, a suspension, a liniment, a lotion, a cream, a drop, an electuary, a spray, an ointment, a patch, a paste, a pill, a suppository and an emulsion, and can contain celluloses (such as methylcellulose), polyhydric alcohols, cyclodextrins (such as hydroxypropyl-β-cyclodextrin), acidic co-solvents (such as citric acid), dendrimers, nanomaterials, sustained-release materials, liposomes or a combination of these substances.

4. The use of one of the preceding claims, wherein the individual with myopia or the individual with a tendency to develop myopia is a child and/or an adolescent, preferably a person aged 3 to 26 years old, more preferably a person aged 6 to 18 years old; or a juvenile, preferably a person whose eyes (eyeballs) are still at the growth and development stages; or school-age people, preferably a student in grades 1 to 12; or people whose parents are highly myopic; or people with insufficient reserves for distance vision.

5. The use of one of the preceding claims, wherein the myopia is refractive myopia or axial myopia; congenital myopia (myopia is present at birth or before school age), early-onset myopia (under 14 years old), delayed onset myopia (16 to 18 years old), or late-onset myopia (after adulthood); low myopia (mild myopia), moderate myopia, or high myopia (severe myopia); pseudomyopia or true myopia; myopia in children and/or adolescents (preferably, aged 3-26 years old, more preferably, aged 6-18 years old), myopia in juveniles, myopia in adults, or myopia in the elderly; simple myopia or pathological myopia; axial simple myopia or simple axial myopia; axial myopia in children and/or adolescents (preferably, aged 3-26 years old, more preferably, aged 6-18 years old); axial myopia in school-age and preschool-age people; primary myopia or secondary myopia; primary myopia in children and/or adolescents (preferably, aged 3-26 years old, more preferably, aged 6-18 years old); or progressive myopia in children and/or adolescents (preferably, aged 3-26 years old, more preferably, aged 6-18 years old); curvature myopia, index myopia, astigmatic myopia, position myopia, or bending myopia; axial myopia in which the refraction continuously changes towards negative; myopia caused by long-term use of eyes at close range, myopia and pseudomyopia caused by visual fatigue, refraction change towards negative caused by adverse drug reactions, myopic eye, myopia caused by reading books, myopia caused by using electronic products such as mobile phones, myopia caused by mismatch of refractive media (components), refractive myopia, myopia caused by abnormal refractive development, myopia caused by excessive eyeball growth, myopia caused by unhygienic use of eyes, the imaging focus of distant objects falling in front of the retina due to various reasons, myopia that is poorly or ineffectively treated with atropine, myopia caused by insufficient outdoor exercise, accommodative spastic myopia, myopia in children, myopia in infants, hereditary myopia, or myopia dominated by environmental factors.

6. The use of one of the preceding claims, wherein the related symptoms of myopia include complications caused by myopia, such as complications of high myopia, and for example, floaters, glaucoma, posterior scleral staphyloma, retinal detachment, retinal tear, amblyopia, macular hemorrhage, choroidal neovascularization, choroidal atrophy, macular degeneration or maculopathy, visual field defect, progressive or sudden decrease in vision (especially near vision), eye soreness and/or pain, night blindness, astigmatism, anisometropia, blindness, vitreous liquefaction, vitreous opacity, squint, frequent blinking, frequent eye rubbing, anisometropia, blurred vision when looking at distant objects, need to squint or partially close eyelids to see distant objects clearly, headache caused by eye strain, inattention caused by myopia, difficulty seeing while driving, especially at night (nocturnal myopia), retinal atrophy and degeneration (hemorrhages and holes), subretinal neovascularization, or eyeball atrophy.

7. The use of one of the preceding claims, wherein the drug, preparation, composition or device further comprises other drugs or ophthalmic preparations, including but not limited to drugs for myopia treatment (such as pirenzepine, muscarinic antagonist, robaxin, indoramine, timolol maleate, epinephrine, pyrazine, perlapine, methylamine, chlorisondamine, acetylcholinesterase inhibitor, dopamine agonist, gamma-aminobutyric acid, naloxone, glucagon, retinoic acid, salidroside, formononetin, etc.), M receptor blockers (such as blockers or antagonists or inhibitors targeting M2 or M3 receptors), bendazac and various salt forms thereof (bendazac lysine), atropine, dibazole, polyunsaturated fatty acids (such as DHA, EPA), prazosin, homatropine, anisodamine (racemic), tropicamide, 7-methylxanthine, niacin, piracetam, Salvia miltiorrhiza extract, safflower extract, fish oil, bear bile extract, vitamins, adenosine triphosphate (ATP), smooth muscle relaxants, drugs to prevent vasospasm, non-selective adenylate antagonist, vasodilators, pupil dilating components, decongestants, eye muscle (e.g., ciliary muscle) accommodative components, anti-inflammatory (antiphlogistic) agent components, astringent components, antihistamine components, anti-allergic components, components for inhibiting collagen degradation, hepatoprotective (avoiding or reducing liver toxicity) components, components for enhancing blood-retinal barrier (making it more difficult for compounds to penetrate this physiological barrier), amino acids, antibacterial agent components, antioxidant components (such as vitamin C, tea polyphenols, glutathione, etc.), saccharides, polymers or derivatives thereof, celluloses or derivatives thereof, local anesthetic components, components for amblyopia treatment, components for glaucoma treatment, components for cataract treatment, type I phosphodiesterase inhibitors (specific or non-specific), miRNA and modified derivatives thereof, therapeutic components for ophthalmic diseases, excipients, *etc.*

8. The use of one of the preceding claims, wherein vinpocetine, or the optical isomer or the racemate, or the solvate, or the pharmaceutically acceptable salt, or the prodrug, or the metabolite, or the related compound or extract, or the crystalline compound thereof, or the combination of these substances, and one or more drugs for prevention and controlling of myopia and/or one or more drugs for treatment of myopia are formulated or designed to be administered continuously, simultaneously, or sequentially, or alternately, or at intervals, or separately.

9. The use of one of the preceding claims, wherein the related compound or extract of vinpocetine is selected from one or more of vinpocetine metabolites (such as apovincaminic acid, hydroxyvinpocetine, hydroxyl-apovincaminic acid and dihydroxy-vinpocetine-glycinate), vinca alkaloid, apovincamine, vinca extract (periwinkle), vincamine, voacanga africana extract, Apocynaceae plant extract, vincristine, tabersonine, vinblastine, vindoline, apovincaminic acid hydrochloride, catharanthine, ethyl vincaminate, methoxyvinpocetine and dihydrovinpocetine.

10. The use of one of the preceding claims, wherein the preparation is a health care product, a food, a dietary supplement, a nutritional product, a drink and other oral products or cosmetics.

11. The use of one of the preceding claims, wherein the device is an instrument, an equipment, a consumable, a system, a medical device, a health care product or an article for changing the appearance of eyes, such as a corneal contact lens, glasses, an intraocular lens, a suture line, an OK lens cleaning (maintenance) system, an eye patch, a patch for improving eyesight, a coloured contact lens, a microneedle, an eye spray system, an eye massager, an eye fumigation device, an ocular surface drug delivery device, an intraocular drug delivery device, a fundus drug delivery device, an implantable pump, a wearable device, an acupoint massager, an eye relaxation device, a myopia treatment device or a drug-device combination for myopia prevention and control.

12. The use of one of the preceding claims, wherein vinpocetine, or the optical isomer or the racemate, or the solvate, or the pharmaceutically acceptable salt, or the prodrug, or the metabolite, or the related compound or extract, or the crystalline compound thereof, or the combination of these substances is used as a sole active ingredient or a main active ingredient or a direct active ingredient; or the content or efficacy of vinpocetine, or the optical isomer or the racemate, or the solvate, or the pharmaceutically acceptable salt, or the prodrug, or the metabolite, or the related compound or extract, or the crystalline compound thereof, or the combination of these substances accounts for 1% or less, or more than 1%, more than 10%, more than 20%, more than 30%, more than 40%, more than 50%, more than 60%, more than 70%, more than 80%, more than 90% or 100% of all active ingredients in the use, and the percentage (%) can be a mass ratio, a molar ratio, or a titer ratio, or an efficacy contribution ratio to the use.

13. The use of one of the preceding claims, wherein the concentration of vinpocetine, or the optical isomer or the racemate, or the solvate, or the pharmaceutically acceptable salt, or the prodrug, or the metabolite, or the related compound or extract, or the crystalline compound thereof, or the combination of these substances in the drug, preparation, composition or device is 0.001 µM to 100 mM, preferably 0.005 µM to 50 mM, preferably 0.01 µM to 1000 µM, preferably 0.05 µM to 100 µM, more preferably 0.1 µM to 25 µM, more preferably 0.1 µM to 15 µM; or the concentration or proportion of these substances or their combination in the drug, preparation, composition or device is less than 25%, preferably less than 5%, preferably less than 1%, more preferably less than 0.01%, more preferably less than 0.001%, and the percentage (%) can be mass/volume concentration (g/100 ml) or mass percentage or mole (number) ratio.

14. A drug, preparation, composition or device which is locally administrated for treating myopia or controlling myopia progression, wherein the concentration of vinpocetine, or the optical isomer or the racemate, or the solvate, or the pharmaceutically acceptable salt, or the prodrug, or the metabolite, or the related compound or extract, or the crystalline compound thereof, or the combination of these substances in the drug, preparation, composition or device is 0.001 µM to 50 mM, preferably 0.01 µM to 1000 µM, preferably 0.05 µM to 100 µM , more preferably 0.1 µM to 25 µM, more preferably 0.1 µM to 15 µM; or the concentration or proportion of these substances or their combination in the drug, preparation, composition or device is less than 25%, preferably less than 5%, preferably less than 1%, more preferably less than 0.01%, more preferably less than 0.001%, and the percentage (%) can be mass/volume concentration (g/100 ml) or mass percentage or mole (number) ratio.

15. A pharmaceutical composition or compound preparation containing at least 2 active substances, wherein the at least 2 active substances comprise vinpocetine, or an optical isomer or a racemate, or a solvate, or a pharmaceutically acceptable salt, or a prodrug, or a metabolite, or a related compound or extract, or a crystalline compound thereof, or a combination of these substances, and other active substances for treating myopia.

16. The pharmaceutical composition or compound preparation of claim 15, wherein the added amount, concentration and/or efficacy of vinpocetine, or the optical isomer or the racemate, or the solvate, or the pharmaceutically acceptable salt, or the prodrug, or the metabolite, or the related compound or extract, or the crystalline compound thereof, or the combination of these substances is not lower than the added amount, concentration and/or efficacy of any other active substances for treating myopia, and the efficacy is aimed at treating myopia or controlling myopia progress.

17. An ocular preparation, wherein, in the preparation, vinpocetine, or the optical isomer or the racemate, or the solvate, or the pharmaceutically acceptable salt, or the prodrug, or the metabolite, or the related compound or extract, or the crystalline compound thereof, or the combination of these substances is a direct, sole or main active ingredient for treating myopia; among them, the concentration of vinpocetine, or the optical isomer or the racemate, or the solvate, or the pharmaceutically acceptable salt, or the prodrug, or the metabolite, or the related compound or extract, or the crystalline compound thereof, or the combination of these substances is 0.001 µM to 50 mM, preferably 0.01 µM to 1000 µM, preferably 0.05 µM to 100 µM , more preferably 0.1 µM to 25 µM, more preferably 0.1 µM to 15 µM; or the concentration or proportion of vinpocetine, or the optical isomer or the racemate, or the solvate, or the pharmaceutically acceptable salt, or the prodrug, or the metabolite, or the related compound or extract, or the crystalline compound thereof, or the combination of these substances is less than 25%, preferably less than 5%, preferably less than 1%, more preferably less than 0.01%, more preferably less than 0.001%, and the percentage (%) can be mass/volume concentration (g/100 ml) or mass percentage or mole (number) ratio.

18. The preparation of claim 17, wherein the preparation includes, but not limited to, an eye drop (eyewash), an eye ointment, an eye spray, an implant tablet, an eye gel, an eye patch, an eye microsphere, an eye sustained-release preparation, a periorbital injection, or an intraocular injection.
